(19) Europäisches Patentamt
European Patent Office
Office européen des brevets

(11) Veröffentlichungsnummer: **0 552 605 A1**

(12) **EUROPÄISCHE PATENTANMELDUNG**

(21) Anmeldenummer: **93100120.0**

(22) Anmeldetag: **07.01.93**

(51) Int. Cl.[5]: **C07C 317/28**, C07C 317/32, C07C 317/40, C07D 295/18

(30) Priorität: **23.01.92 DE 4201699**

(43) Veröffentlichungstag der Anmeldung: **28.07.93 Patentblatt 93/30**

(84) Benannte Vertragsstaaten: **CH DE FR GB IT LI**

(71) Anmelder: **BASF Aktiengesellschaft**
**Carl-Bosch-Strasse 38**
**W-6700 Ludwigshafen(DE)**

(72) Erfinder: **Siegel, Bernd, Dr.**
**Faberstrasse 39**
**W-6700 Ludwigshafen(DE)**
Erfinder: **Patsch, Manfred, Dr.**
**Fritz-Wendel-Strasse 4**
**W-6706 Wachenheim(DE)**
Erfinder: **Kessel, Knut, Dr.**
**Berliner Strasse 18**
**W-6800 Mannheim 1(DE)**

(54) **Gemischte Oxalsäurediamide.**

(57) Oxalsäurederivate der Formel

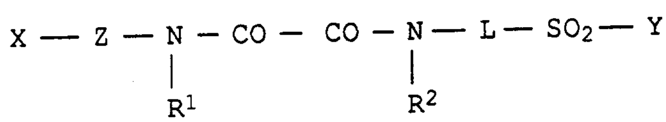

,

in der

- $R^1$ und $R^2$ — Wasserstoff, $C_1$-$C_4$-Alkyl oder Phenyl,
- X — Hydroxy, Nitro oder einen Rest der Formel $-NR^3R^4$, worin $R^3$ für Wasserstoff oder $C_1$-$C_4$-Alkanoyl und $R^4$ für Wasserstoff, $C_1$-$C_4$-Alkyl oder Phenyl stehen,
- Z — $C_2$-$C_8$-Alkylen, gegebenenfalls substituiertes Phenylen, gegebenenfalls substituiertes Naphthylen oder X-Z und $R^1$ zusammen mit dem sie verbindenden Stickstoffatom den Rest der Formel

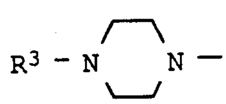

,

worin $R^3$ die obengenannte Bedeutung besitzt,
- L — ein Brückenglied und
- Y — Vinyl oder einen Rest der Formel $-C_2H_4$-A, worin A für Hydroxy oder eine unter alkalischen Reaktionsbedingungen abspaltbare Gruppe steht, bedeuten,

sowie ihre Verwendung als Zwischenprodukte für die Herstellung von Farbstoffen.

EP 0 552 605 A1

Die vorliegende Erfindung betrifft neue Oxalsäurediamide der Formel I

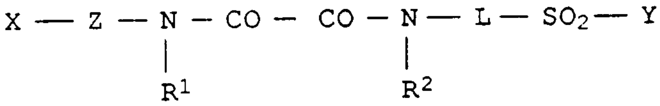 (I),

in der

| | |
|---|---|
| $R^1$ und $R^2$ | unabhängig voneinander jeweils Wasserstoff, $C_1$-$C_4$-Alkyl oder Phenyl, |
| X | Hydroxy, Nitro oder einen Rest der Formel $-NR^3R^4$, worin $R^3$ für Wasserstoff oder $C_1$-$C_4$-Alkanoyl und $R^4$ für Wasserstoff, $C_1$-$C_4$-Alkyl oder Phenyl stehen, |
| Z | $C_2$-$C_8$-Alkylen, gegebenenfalls substituiertes Phenylen, gegebenenfalls substituiertes Naphthylen oder X-Z und $R^1$ zusammen mit dem sie verbindenden Stickstoffatom den Rest der Formel 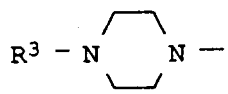 , worin $R^3$ die obengenannte Bedeutung besitzt, |
| L | $C_2$-$C_8$-Alkylen, einen Rest der Formel 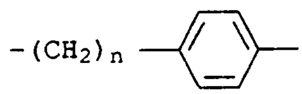 oder 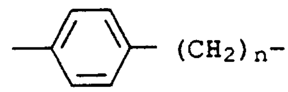 worin n für 1 oder 2 steht, gegebenenfalls substituiertes Phenylen oder Naphthylen und |
| Y | Vinyl oder einen Rest der Formel $-C_2H_4$-A, worin A für Hydroxy oder eine unter alkalischen Reaktionsbedingungen abspaltbare Gruppe steht, bedeuten, |

sowie ihre Verwendung als Zwischenprodukte für die Herstellung von Farbstoffen.

Aufgabe der vorliegenden Erfindung war es, neue gemischte Oxalsäurediamide bereitzustellen, die über ungesättigte Reste oder deren Vorläufer verfügen und die sich vorteilhaft als Anker für Reaktivfarbstoffe und, für den Fall, daß sie zusätzlich eine unsubstituierte Aminogruppe aufweisen, auch als Diazokomponente oder Kupplungskomponente eignen sollten.

Demgemäß wurden die eingangs näher bezeichneten Oxalsäurediamide der Formel I gefunden.

Alle in der obengenannten Formel I auftretenden Alkyl- und Alkylengruppen können sowohl geradkettig als auch verzweigt sein.

Wenn in der obengenannten Formel I substituierte Phenylen- oder Naphthylenreste auftreten, so können als Substituenten beispielsweise $C_1$-$C_4$-Alkyl, $C_1$-$C_4$-Alkoxy, Halogen, Carboxyl oder Hydroxysulfonyl in Betracht kommen. Die Phenylen- oder Naphthylenreste sind dabei üblicherweise ein-bis dreifach substituiert.

Reste $R^1$, $R^2$ und $R^4$ sind z.B. Methyl, Ethyl, Propyl, Isopropyl, Butyl, Isobutyl und sec-Butyl.

Reste $R^3$ sind z.B. Formyl, Acetyl, Propionyl, Butyryl oder Isobutyryl.

Reste Z und L sind z.B. $-(CH_2)_2-$, $-(CH_2)_3-$, $-(CH_2)_4-$, $-(CH_2)_5-$, $-(CH_2)_6-$, $-(CH_2)_7-$, $-(CH_2)_8-$, $-CH(CH_3)-CH_2-$, $-CH(CH_3)-CH(CH_3)-$, 1,2-, 1,3- oder 1,4-Phenylen, 2- oder 3-Methyl-1,4-phenylen, 2- oder 3-Methoxy-1,4-phenylen, 2- oder 3-Chlor-1,4-phenylen, 2- oder 3-Hydroxysulfonyl-1,4-phenylen, 2-, 3- oder 4-Hydroxysulfonyl-1,3‾phenylen, 2,5-Dimethyl-1,4-phenylen, 2,6-Dimethyl-1,4-phenylen, 2,5-Dimethoxy-1,4-phenylen, 2,6-Dimethoxy-1,4-phenylen, 2- oder 3-Carboxyl-1,4-phenylen oder 2- oder 4-Carboxyl-1,3-phenylen.

Reste L sind weiterhin z.B. 1,4- oder 1,8-Naphthylen,

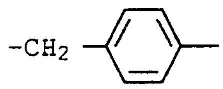, 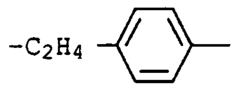, 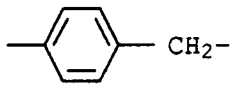 oder 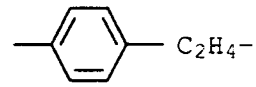

Wenn Y in Formel I den Rest $-C_2H_4-A$ bedeutet, steht A u.a. für eine unter alkalischen Reaktionsbedingungen abspaltbare Gruppe. Solche Gruppen sind z.B. Chlor, $OSO_3H$, $SSO_3H$, $OP(O)(OH)_2$, $C_1-C_4$-Alkylsulfonyloxy, gegebenenfalls substituiertes Phenylsulfonyloxy, $C_1-C_4$-Alkanoyloxy, $C_1-C_4$-Dialkylamino oder ein Rest der Formel

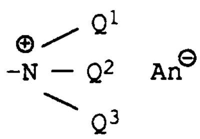, 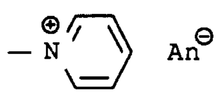, 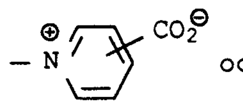 oder 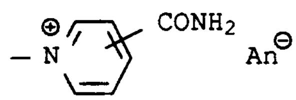,

wobei $Q^1$, $Q^2$ oder $Q^3$ unabhängig voneinander jeweils die Bedeutung von $C_1-C_4$-Alkyl oder Benzyl und $An^{\ominus}$ jeweils die Bedeutung des Äquivalents eines Anions besitzen. (Geeignete Anionen sind z.B. Fluorid, Chlorid, Bromid, Iodid, Mono-, Di- oder Trichloracetat, Methylsulfonat, Phenylsulfonat oder 2- oder 4-Methylphenylsulfonat.)

Bevorzugt sind Oxalsäurediamide der Formel I, in der X Hydroxy oder Amino bedeutet.

Bevorzugt sind weiterhin Oxalsäurediamide der Formel I, in der Z und L unabhängig voneinander jeweils Phenylen oder $C_2-C_6$-Alkylen bedeuten.

Bevorzugt sind weiterhin Oxalsäurediamide der Formel I, in der Y Vinyl oder einen Rest der Formel $-C_2H_4-A$ bedeutet, worin A für eine unter alkalischen Reaktionsbedingungen abspaltbare Gruppe steht.

Besonders bevorzugt sind Oxalsäurediamide der Formel I, in der X Amino bedeutet.

Besonders bevorzugt sind weiterhin Oxalsäurediamide der Formel I, in der Z Phenylen bedeutet.

Die erfindungsgemäßen Oxalsäurediamide können nach an sich bekannten Methoden erhalten werden. Beispielsweise kann man in einem ersten Schritt ein Oxalsäurederivat der Formel II

$B^1O-CO-CO-B^2$ (II),

in der $B^1$ $C_1-C_4$-Alkyl und $B^2$ $C_1-C_4$-Alkoxy oder Halogen bedeuten, mit einem Amin der Formel III

$X^1-Z-NH-R^1$ (III),

in der $X^1$ Hydroxy, Nitro oder gegebenenfalls durch $C_1-C_4$-Alkyl oder Phenyl substituiertes $C_1-C_4$-Alkanoylamino bedeutet und $R^1$ und Z jeweils die obengenannte Bedeutung besitzen, zu einem Oxalsäureesteramid der Formel IV

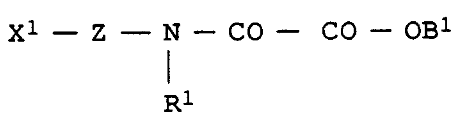 (IV),

in der $B^1$, $R^1$, $X^1$ und Z jeweils die obengenannte Bedeutung besitzen, umsetzen.

In einem zweiten Schritt kann dann die Umsetzung mit einer schwefelhaltigen Aminverbindung der Formel Va oder Vb

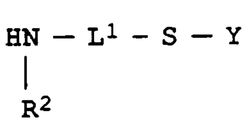

(Va)

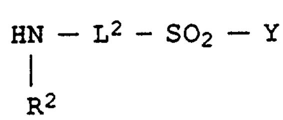

(Vb)

wobei $L^1$ $C_2$-$C_8$-Alkylen und $L^2$ einen Rest der Formel

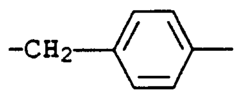 , 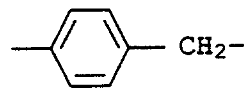 ,

gegebenenfalls substituiertes Phenylen oder Naphthylen bedeuten und $R^2$ und Y jeweils die obengenannte Bedeutung besitzen, erfolgen, wobei man ein Oxalsäurediamid der Formel VI oder Ia

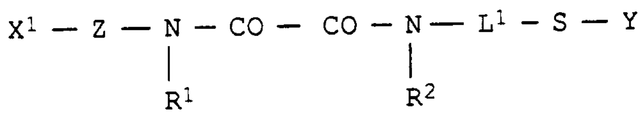
(VI)

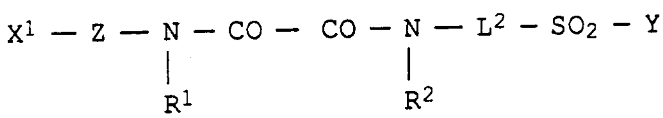
(Ia)

erhält, wobei $X^1$, $R^1$, $R^2$, $L^1$, $L^2$, Y und Z jeweils die obengenannte Bedeutung besitzen.

Im Fall des Oxalsäurediamids VI erfolgt noch eine Oxidation des Thioethers zum Sulfon der Formel Ib

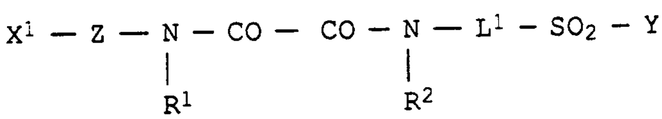
(Ib),

in der $X^1$, $R^1$, $R^2$, $L^1$, Y und Z jeweils die obengenannte Bedeutung besitzen, die z.B. mittels Wasserstoffperoxid oder Hypochlorit vorgenommen werden kann.

Aus den erfindungsgemäßen Verbindungen der Formel Ia oder Ib können die Aminoverbindungen ($X^1$ = Amino oder $C_1$-$C_4$-Alkyl- oder Phenylamino) entweder durch Hydrierung der Nitrogruppe ($X^1$ = Nitro) oder durch Entacyclierung ($X^1$ = acyliertes Amino) erhalten werden.

Es ist auch möglich, die Reihenfolge der ersten beiden Reaktionsschritte umzukehren.

Die neuen Oxalsäurediamide der Formel I sind wertvolle Zwischenprodukte für die Synthese von Farbstoffen.

Insbesondere können diejenigen Sulfonylverbindungen der Formel Ic

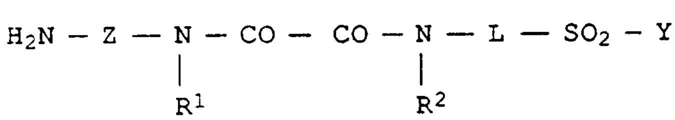
(Ic),

in der Z gegebenenfalls substituiertes Phenylen oder gegebenenfalls substituiertes Naphthylen und Y Vinyl oder einen Rest der Formel $-C_2H_4-A$, worin A für eine unter alkalischen Reaktionsbedingungen abspaltbare Gruppe steht, bedeuten und $R^1$, $R^2$ und L jeweils die obengenannte Bedeutung besitzen, als Ankersystem und gegebenenfalls gleichzeitig als Diazokomponente oder Kupplungskomponente bei der Herstellung von Reaktivfarbstoffen Verwendung finden.

Diejenigen Sulfonylverbindungen der Formel Id

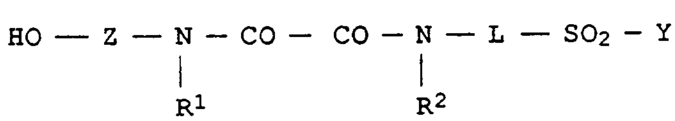
(Id),

in der Y Vinyl oder einen Rest der Formel $-C_2H_4-A$, worin A für eine unter alkalischen Reaktionsbedingungen abspaltbare Gruppe steht, bedeutet und $R^1$ , $R^2$, Z und L jeweils die obengenannte Bedeutung besitzen, können ebenfalls insbesondere als Ankersystem bei der Herstellung von Reaktivfarbstoffen Verwendung finden.

Die folgenden Beispiele sollen die Erfindung näher erläutern.

Beispiel 1.1

Ein Gemisch aus 138 g (1 mol) 3-Nitroanilin und 409 g (2,8 mol) Diethyloxalat wurde auf 150 bis 165 °C erwärmt und der dabei entstehende Alkohol abdestilliert. Nach vollständiger Umsetzung (DC-Kontrolle) wurde das Reaktionsgemisch auf Raumtemperatur abgekühlt, der entstandene Niederschlag abgesaugt und getrocknet. Es resultierten 217 g einer Verbindung der Formel

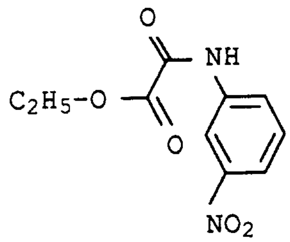

mit dem Schmelzpunkt 153 bis 157 °C.
$^1$H-NMR ($d_6$-DMSO); $\delta$ = 1,37 (t,3H, $CH_3$); 4,37 (q, 2H, $CH_2$); 7,67 (t,1H, Aromaten-H); 8,03 (d,1H, Aromaten-H); 8,17 (d,1H, Aromaten-H); 11,27 (s,1H,NH) ppm.

Beispiel 1.2

138 g (1 mol) 3-Nitroanilin wurden in 1000 ml Wasser suspendiert. Hierzu tropfte man innerhalb von 2 Stunden unter kräftigem Rühren 177,5 g (1,3 mol) Oxalsäureethylesterchlorid und hielt den pH-Wert mittels 2N wäßriger Kaliumhydrogencarbonatlösung bei 4 bis 5. Nach beendeter Umsetzung (DC-Kontrolle) wurde der entstandene Niederschlag abgesaugt und getrocknet. Es verblieben 199 g der in Beispiel 1.1 beschriebenen Verbindung.

In analoger Weise werden die in Tabelle 1 aufgeführten Verbindungen erhalten.

Tabelle 1

| Bsp.-Nr. | Formel | Schmp. |
|---|---|---|
| 1.3 | 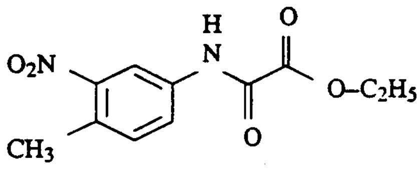 | 122-129°C |
| 1.4 | 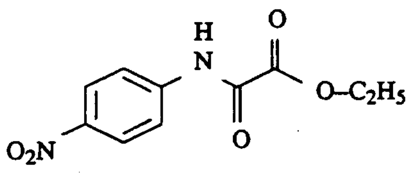 | 175-180°C |
| 1.5 | 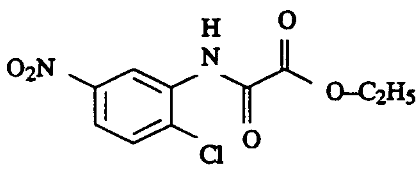 | 105-110°C |
| 1.6 | 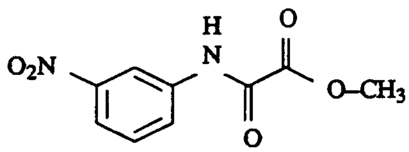 | |
| 1.7 | 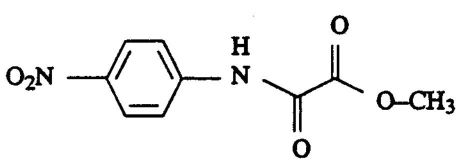 | |
| 1.8 | 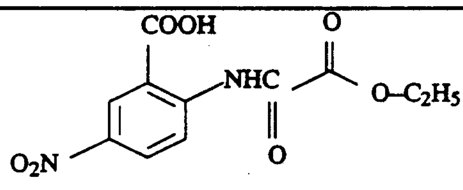 | |

| Bsp.-Nr. | Formel | Schmp. |
|---|---|---|
| 1.9 | 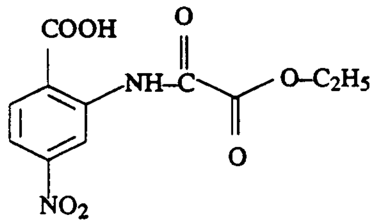 | |
| 1.10 | 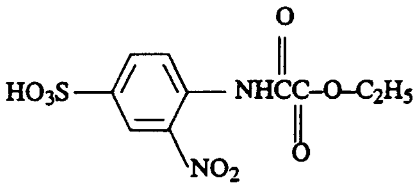 | |
| 1.11 | 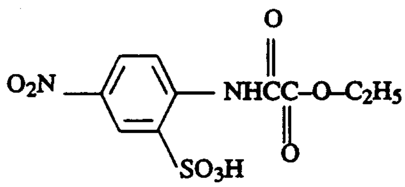 | |
| 1.12 | 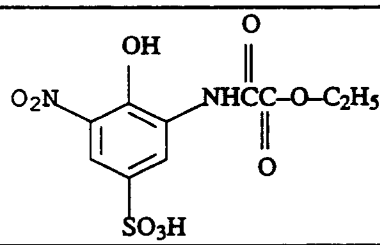 | |
| 1.13 | 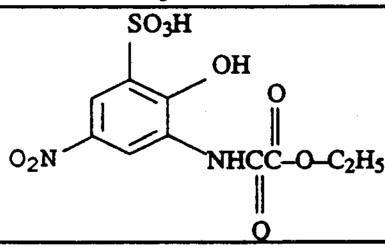 | |

Beispiel 2.1

409 g (2,8 mol) Diethyloxalat und 102 g (1 mol) 1-Acetylamino-2-aminoethan wurden bei Raumtemperatur vorsichtigt vereinigt und anschließend für ca. 1 Stunde auf 90 ° C erhitzt. Nach beendeter Umsetzung wurde das Reaktionsgemisch abgekühlt, der Niederschlag abgesaugt, mit 250 ml Methyl-tert-butylether nachgewaschen und getrocknet. Es wurden 150 g einer Verbindung mit der Formel

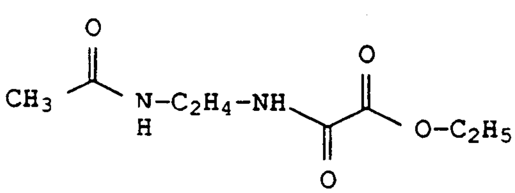

erhalten. Schmelzpunkt 253 bis 256 °C.
$^1$H-NMR ($d_6$-DMSO): $\delta$ = 1,27 (t,3H,$CH_3$); 1,80 (s,3H,$CH_3$); 3,17 (m,4H,$CH_2$-$CH_2$); 4,23 (q,2H,$CH_2$); 7,93 (t,1H,NH); 8,93 (t,1H,NH) ppm.

Analog Beispiel 2.1 werden die in Tabelle 2 aufgeführten Verbindungen erhalten.

Tabelle 2

| Bsp.-Nr. | Formel |
|---|---|
| 2.2 | 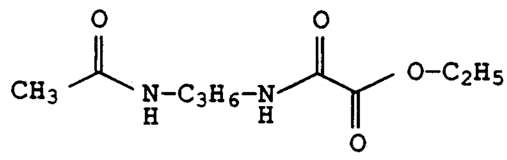 |
| 2.3 | 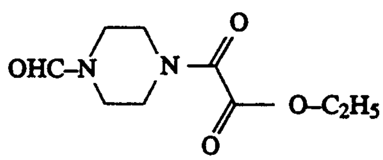 |
| 2.4 | 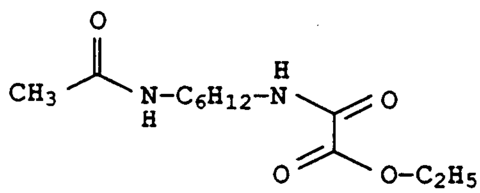 |
| 2.5 | 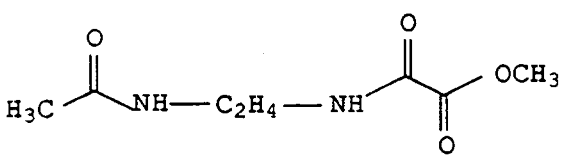 |
| 2.6 | 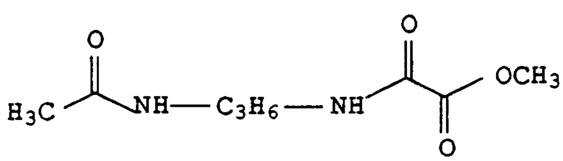 |

Beispiel 3.1

201 g (1 mol) 4-Aminophenyl-2'-hydroxyethylsulfon wurden mit 292 g (2 mol) Diethyloxalat 9 Stunden zum Sieden erhitzt. Nach vollständiger Umsetzung (DC-Kontrolle) wurde das Reaktionsgemisch abgekühlt, mit 500 ml Methyl-tert-butylether versetzt und der entstandene Niederschlag abgesaugt. Nach dem Trocknen verblieben 259 g einer Verbindung der Formel

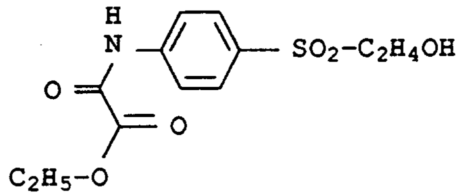

mit dem Schmelzpunkt von 138 bis 143 °C.
$^1$H-NMR ($d_6$-DMSO); $\delta$ = 1,33 (t,3H,$CH_3$); 3,43 (m,2H,$CH_2$); 3,67 (m,2H,$CH_2$); 4,33 (q,2H,$CH_2$); 4,70 (t,1H,OH); 7,88-8,03 (m,4H, Aromaten-H); 11,20 (s,1H,NH) ppm.

Beispiel 3.2

100,5 g (0,5 mol) 4-Aminophenyl-2'-hydroxyethylsulfon wurden in 400 ml Tetrahydrofuran gelöst und mit 50,5 g (0,5 mol) Triethylamin versetzt. Hierzu wurde bei Raumtemperatur langsam ein Gemisch aus 75,1 g (0,55 mol) Oxalsäureethylesterchlorid und 75 ml Tetrahydrofuran zugetropft. Anschließend wurde das Reaktionsgemisch zur Vervollständigung der Umsetzung noch ca. 1,5 Stunden zum Sieden erhitzt, abgekühlt, vom entstandenen Triethylamin-hydrochlorid abgetrennt und die Mutterlauge auf ca. 75 ml eingeengt. Der verbleibende Feststoff wurde isoliert und getrocknet. Es entstandene 121 g der in Beispiel 3.1 beschriebenen Verbindung.

In analoger Weise werden die in der folgenden Tabelle 3 aufgeführten Verbindungen erhalten.

Tabelle 3

| Bsp.-Nr. | Formel |
|---|---|
| 3.3 | 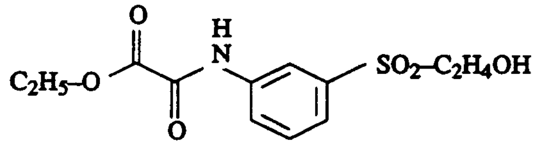 |
| 3.4 | 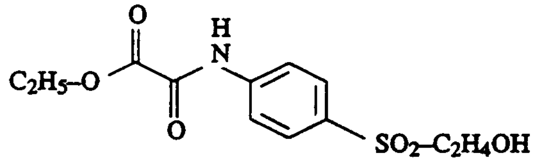 |
| 3.5 | 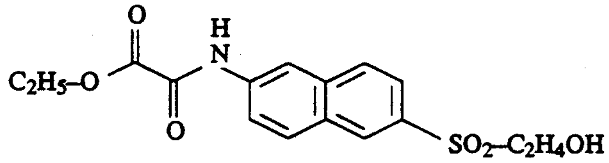 |
| 3.6 | 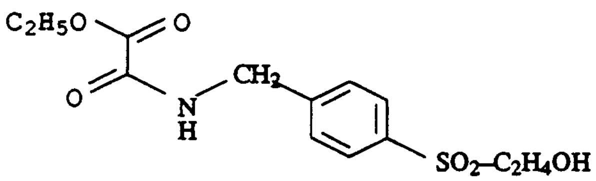 |

| Bsp.-Nr. | Formel |
|---|---|
| 3.7 | 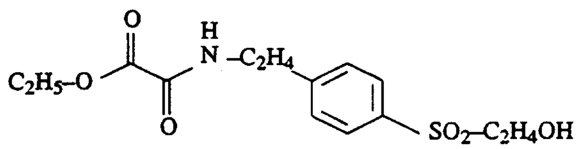 |
| 3.8 | 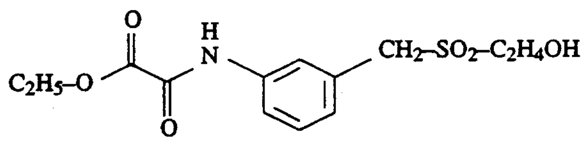 |
| 3.9 | 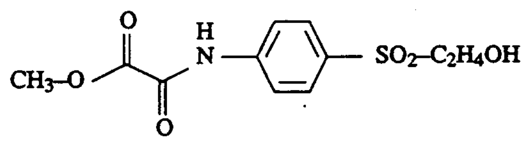 |
| 3.10 | 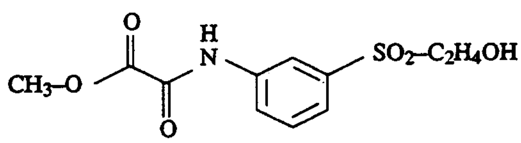 |
| 3.11 | 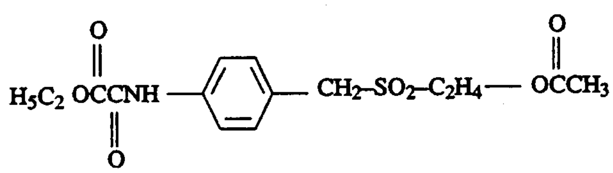 |
| 3.12 | 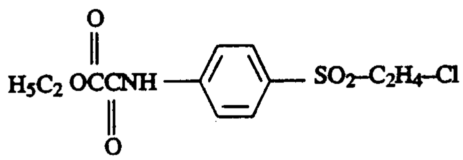 |
| 3.13 | 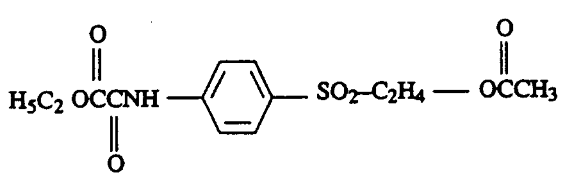 |

| Bsp.-Nr. | Formel |
| --- | --- |
| 3.14 | 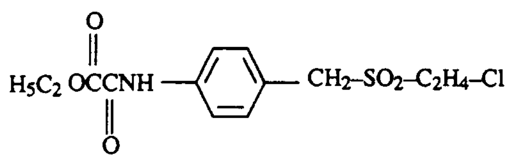 |
| 3.15 | 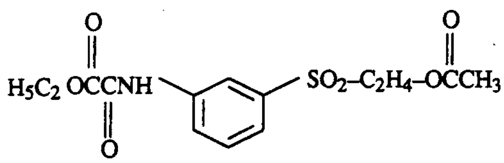 |
| 3.16 | 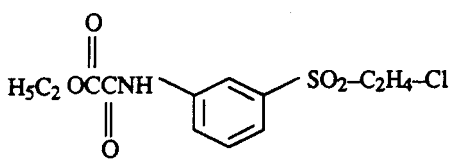 |
| 3.17 | 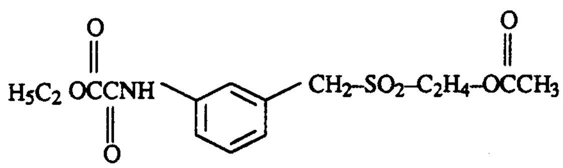 |
| 3.18 | 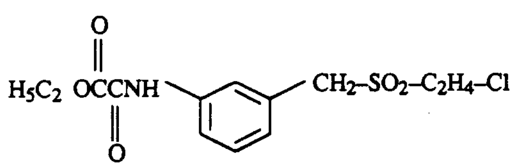 |
| 3.19 | 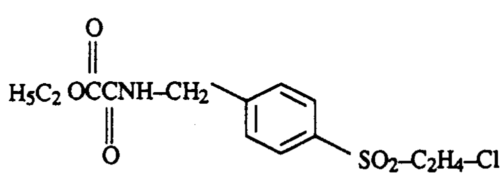 |
| 3.20 | 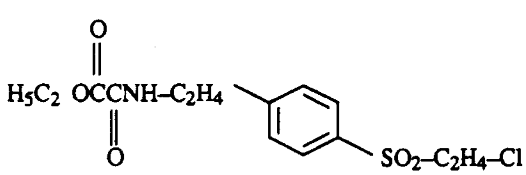 |

Beispiel 4.1

238 g (1 mol) der in Beispiel 1.1 beschriebenen Verbindung wurden mit 123 g (1,02 mol) 2-Aminoethyl-2'-hydroxyethylsulfid und 1000 ml Toluol für 6 Stunden auf 100 ° C erhitzt. Nach beendeter Umsetzung (DC-Kontrolle) wurde das Reaktionsgemisch abgekühlt, der Niederschlag abgesaugt und getrocknet. Es entstanden 310 g einer Verbindung der Formel

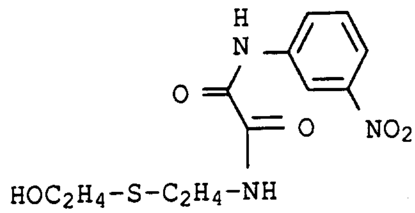

mit einem Schmelzpunkt von 108 bis 118 °C.
$^1$H-NMR ($d_6$-DMSO): $\delta$ = 2,60-2,67 (m,4H, 2 $CH_2$); 3,35-3,60 (m,4H, 2 $CH_2$); 4,87 (t,1H,OH); 7,65 (t,1H, Aromaten-H); 8,02-8,25 (m,2H, Aromaten-H); 8,85 (t,1H, Aromaten-H); 9,17 (t,1H,NH); 11,17 (s,1H,NH) ppm.

In analoger Weise werden die in der folgenden Tabelle 4 aufgeführten Verbindungen erhalten. (Bei denjenigen Verbindungen, die Carboxyl- oder Hydroxysulfonylgruppen aufweisen, wurde anstelle von Toluol N,N-Dimethylformamid als Reaktionsmedium verwendet).

Tabelle 4

| Bsp.-Nr. | Formel | Schmp. |
|---|---|---|
| 4.2 | 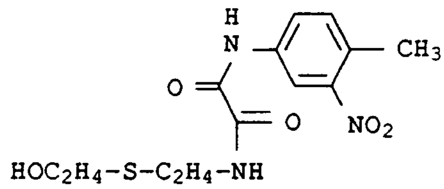 | 133-140°C |
| 4.3 | 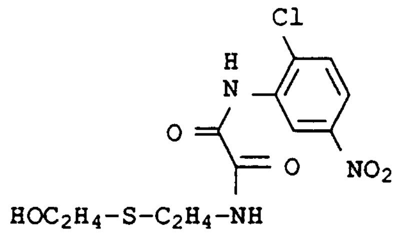 | 119-121°C |
| 4.4 | 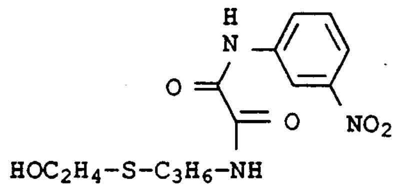 | 110-115°C |
| 4.5 | 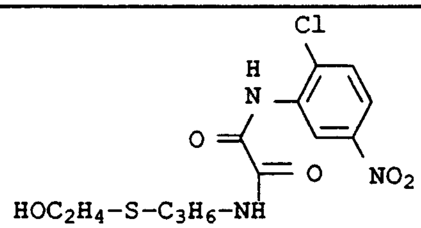 | 160-165°C |
| 4.6 | 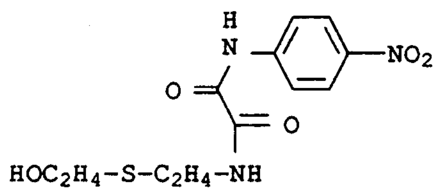 | 143-151°C |
| 4.7 | 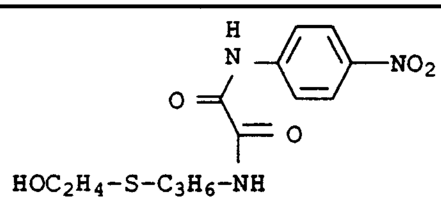 | 138-145°C |

| Bsp.-Nr. | Formel | Schmp. |
|---|---|---|
| 4.8 | 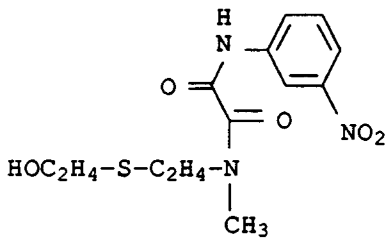 | |
| 4.9 | 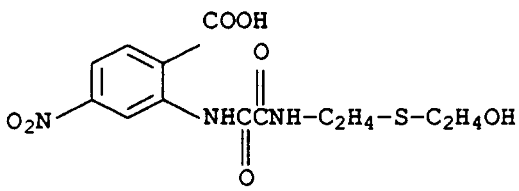 | |
| 4.10 | 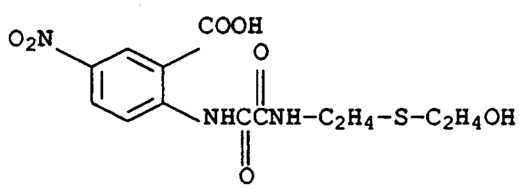 | |
| 4.11 | 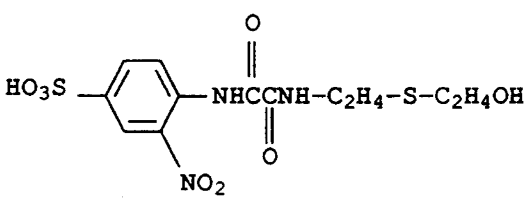 | |
| 4.12 | 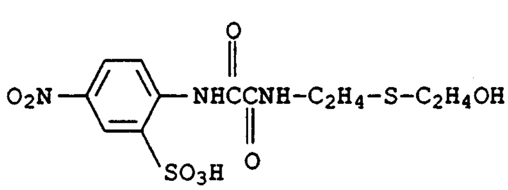 | |
| 4.13 | 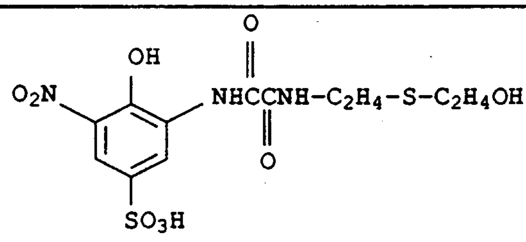 | |

| Bsp.-Nr. | Formel | Schmp. |
|---|---|---|
| 4.14 | 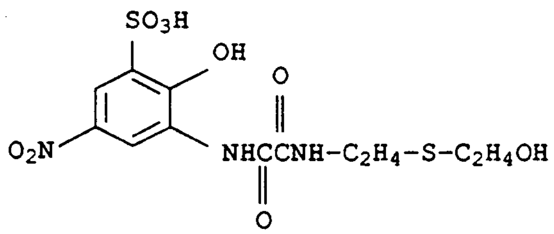 | |
| 4.15 | 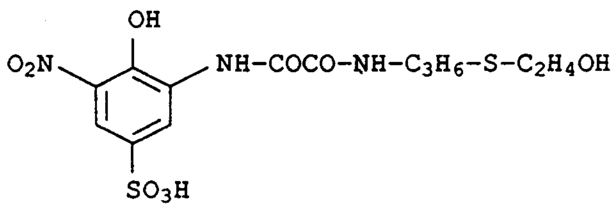 | |

Beispiel 5.1

202 g (1 mol) der in Beispiel 2.1 beschriebenen Verbindung wurden mit 201 g (1 mol) 3-Aminophenyl-2-hydroxyethylsulfon in 500 ml N,N-Dimethylformamid für 7 Stunden auf 100 °C erwärmt. Nach beendeter Reaktion wurde das Lösungsmittel weitestgehend abgezogen und der Rückstand mit 1000 ml Wasser verrührt. Der entstandene Niederschlag wurde abgesaugt und getrocknet. Es verblieben 303 g einer Verbindung der Formel

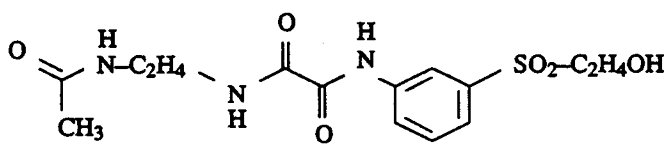

mit einem Schmelzpunkt von 177 bis 181 °C.
$^1$H-NMR ($d_6$-DMSO): $\delta$ = 1,88 (s,3H,$CH_3$); 3,30 (m,2H,$CH_2$), 3,35 (m,2H,$CH_2$); 3,50 (t,2H,$CH_2$); 3,78 (t,2H,$CH_2$); 3,97 (s,1H,OH); 7,65-7,73 (m,2H, Aromaten-H); 8,03(t,1H,NH); 8,17 (d,1H, Aromaten-H); 8,55 (s,1H, Aromaten-H); 9,05 (t,1H,NH); 11,00 (s,1H,NH) ppm.

Beispiel 5.2

102 g (1 mol) 1-Acetylamino-2-aminoethan und 301 g (1 mol) der in Beispiel 3.3 beschriebenen Verbindung wurden in 500 ml N,N-Dimethylformamid für 7 Stunden auf 100 °C erhitzt. Nach vollständiger Umsetzung (DC-Kontrolle) wurde das Lösungsmittel weitestgehend abgezogen und der Rückstand mit 500 ml Wasser versetzt. Der entstandene Niederschlag wurde abgesaugt und getrocknet. Es resultierten 300 g der in Beispiel 5.1 beschriebenen Verbindung.

In analoger Weise werden die in der folgenden Tabelle 5 aufgeführten Verbindungen erhalten.

Tabelle 5

| Bsp.-Nr. | Formel |
|---|---|
| 5.3 | 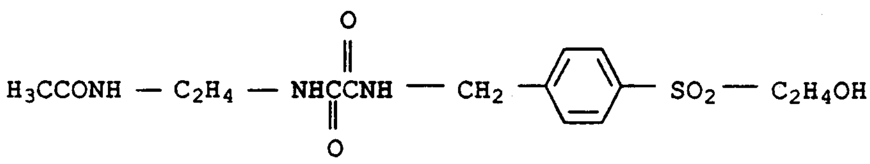 |
| 5.4 | 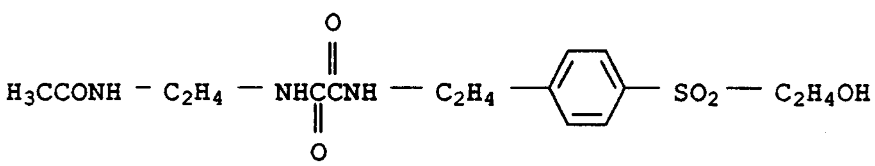 |
| 5.5 | 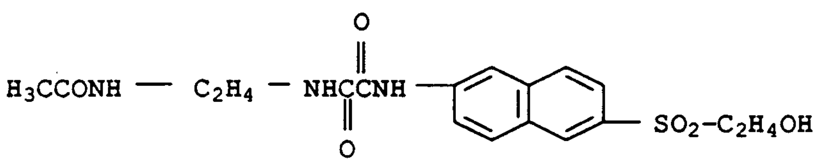 |
| 5.6 | 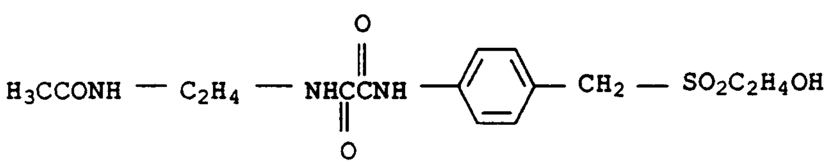 |
| 5.7 | 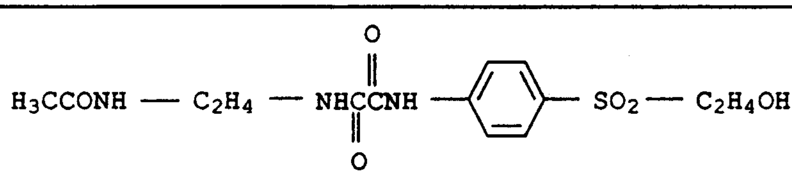 |

| Bsp.-Nr. | Formel |
|---|---|
| 5.8 | 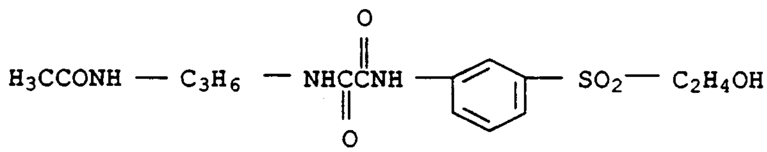 |
| 5.9 | 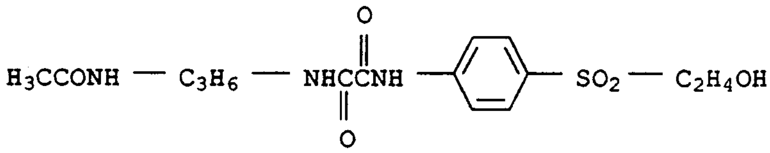 |
| 5.10 | 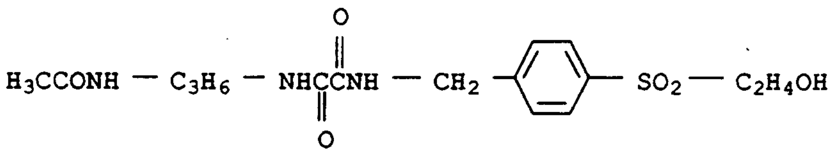 |
| 5.11 | 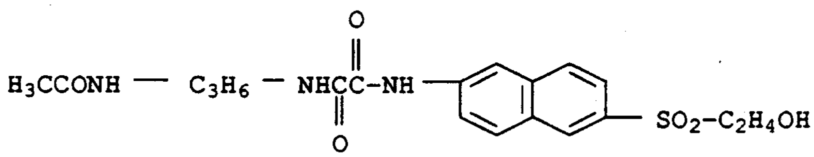 |
| 5.12 | 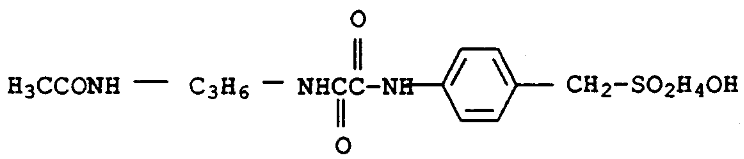 |
| 5.13 | 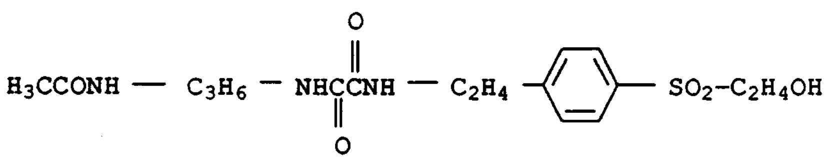 |
| 5.14 | 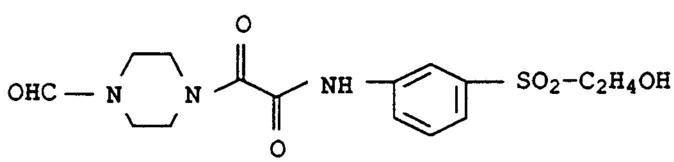 |
| 5.15 | 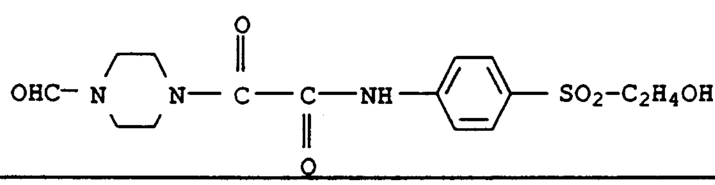 |

| Bsp.-Nr. | Formel |
|---|---|
| 5.16 | 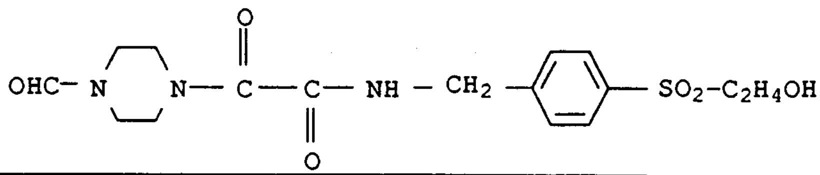 |
| 5.17 | 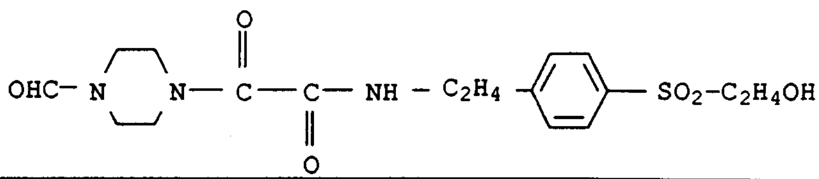 |
| 5.18 | 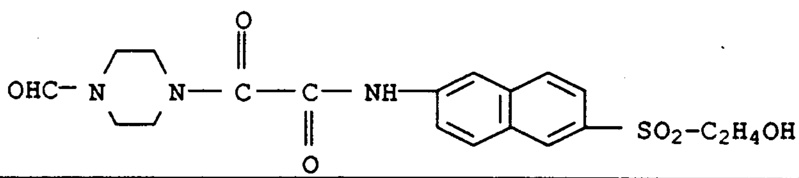 |
| 5.19 | 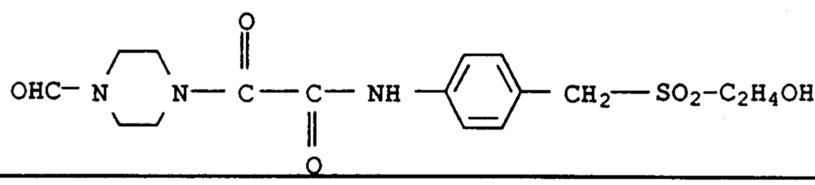 |

Beispiel 6.1

238 g (1 mol) der in Beispiel 1.1 beschriebenen Verbindung und 201 g (1 mol) 3-Aminophenyl-2'-hydroxyethylsulfon wurden in 500 ml N,N-Dimethylformamid für 7 Stunden auf 100 °C erhitzt. Nach Ende der Reaktion (DC-Kontrolle) wurde das Lösungsmittel weitestgehend abgezogen und der Rückstand mit 500 ml Wasser versetzt. Der entstandene Niederschlag wurde abgesaugt und getrocknet. Es verblieben 334 g einer Verbindung der Formel

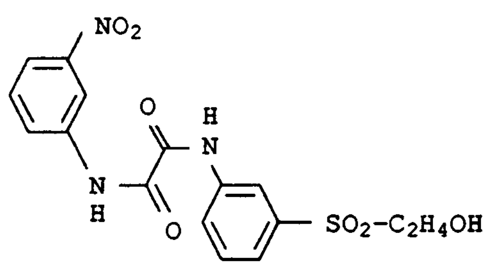

$^1$H-NMR ($d_6$-DMSO); $\delta$ ) = 3,48 (t,2H, $CH_2$); 3,77 (t,2H,$CH_2$); 4,65 (s,1H,OH); 7,73 (m,3H, Aromaten-H); 8,03-8,32 (m,3H, Aromaten-H); 8,58 (s,1H, Aromaten-H); 8,92 (s,1H, Aromaten-H); 11,32 (s,1H,NH); 11,42 (s,1H,NH) ppm.

Beispiel 6.2

138 g (1 mol) 3-Nitroanilin wurden mit 301 g (1 mol) der in Beispiel 3.3 beschriebenen Verbindung in 500 ml N,N-Dimethylformamid für 7 Stunden auf 100 °C erhitzt. Nach vollständiger Umsetzung (DC-Kontrolle) wurde das Lösungsmittel weitestgehend abgezogen und der Rückstand mit 500 ml Wasser verdünnt. Der entstandene Niederschlag wurde abgesaugt, gewaschen und getrocknet. Es verblieben 330 g der in Beispiel 6.1 beschriebenen Verbindung.

In analoger Weise werden die in der folgenden Tabelle 6 aufgeführten Verbindungen erhalten.

Tabelle 6

| Bsp.-Nr. | Formel |
|---|---|
| 6.3 | 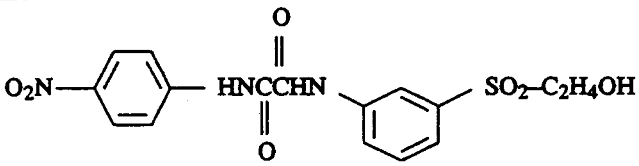 |
| 6.4 | 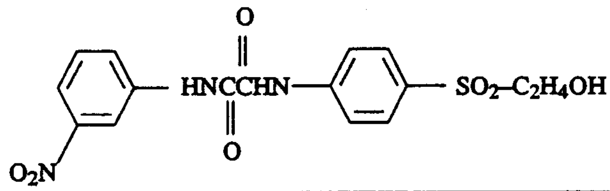 |
| 6.5 | 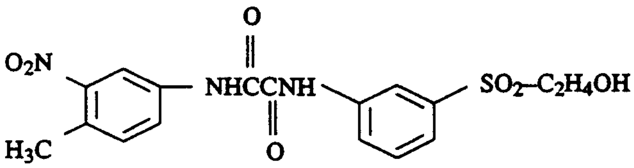 |
| 6.6 | 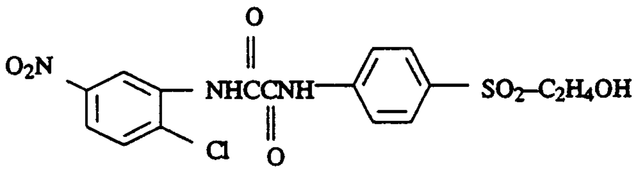 |

| Bsp.-Nr. | Formel |
|---|---|
| 6.7 | 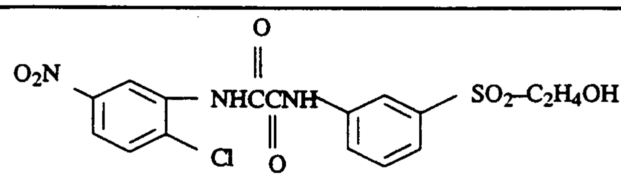 |
| 6.8 | 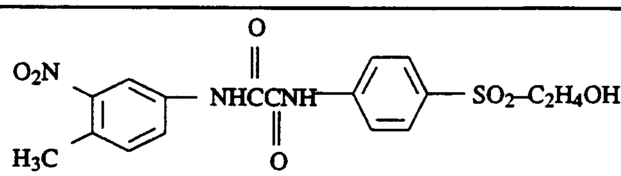 |
| 6.9 | 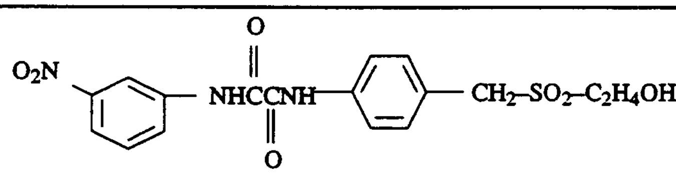 |
| 6.10 | 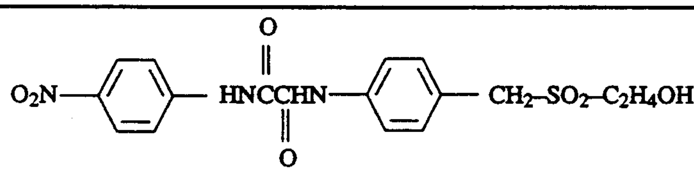 |
| 6.11 | 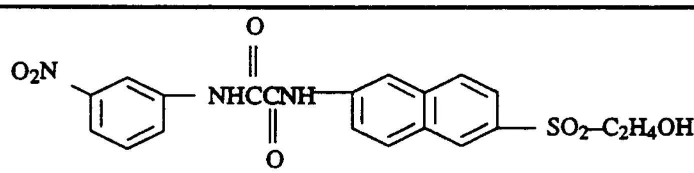 |
| 6.12 | 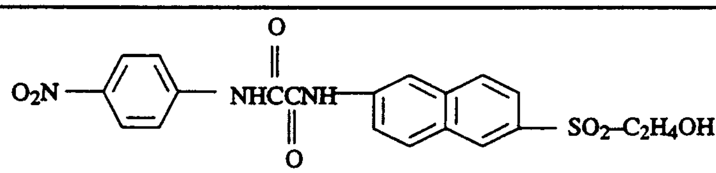 |
| 6.13 | 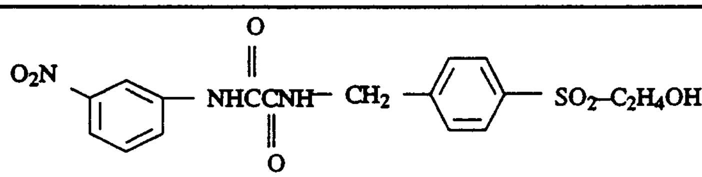 |
| 6.14 | 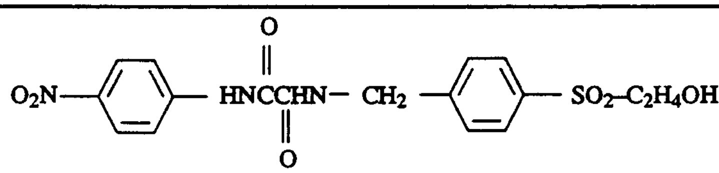 |

| Bsp.-Nr. | Formel |
|---|---|
| 6.15 | 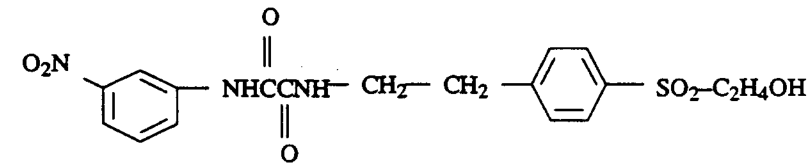 |
| 6.16 | 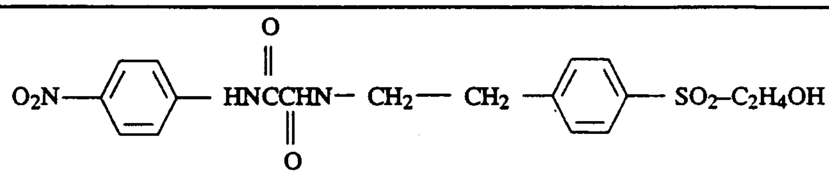 |
| 6.17 | 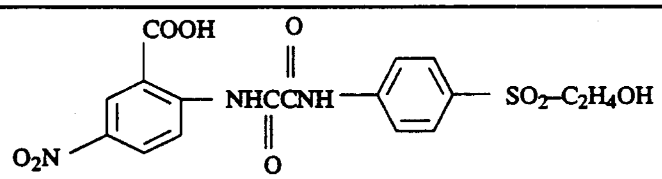 |
| 6.18 | 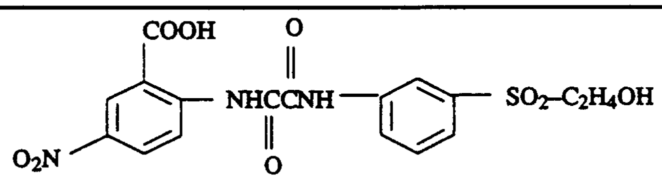 |
| 6.19 | 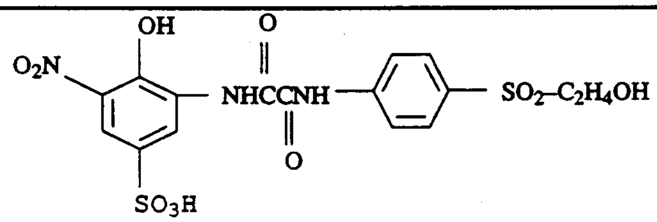 |
| 6.20 | 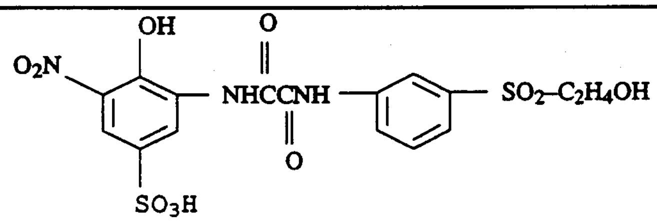 |

Beispiel 7.1

202 g (1 mol) der in Beispiel 2.1 beschriebenen Verbindungen wurden mit 121 g (1 mol) 2-Aminoethyl-2'-hydroxyethylsulfid in 1000 ml Tetrahydrofuran für 5 Stunden zum Sieden erhitzt. Anschließend wurde das Reaktionsgemisch auf ca. 10 °C abgekühlt und der entstandene Niederschlag abgesaugt. Es entstanden 240 g einer Verbindung der Formel

$$CH_3CO - NH - C_2H_4 - NH - \overset{\overset{O}{\|}}{C} - \underset{\underset{O}{\|}}{C} - NH - C_2H_4 - S - C_2H_4OH$$

$^1H$-NMR ($d_6$-DMSO); δ = 1,80 (s,3H, $CH_3$); 2,60 (m,4H,$CH_2$); 3,18 (m,4H,$CH_2$); 3,32 (m,2H,$CH_2$); 3,55 (t,2H,$CH_2$); 4,75 (s,1H,OH); 7,97 (t,1H,NH); 8,80 (t,1H,NH); 8,87 (t,1H,NH) ppm.

In analoger Weise werden die in der folgenden Tabelle 7 aufgeführten Verbindungen erhalten.

Tabelle 7

| Bsp.-Nr. | Formel |
|---|---|
| 7.2 | 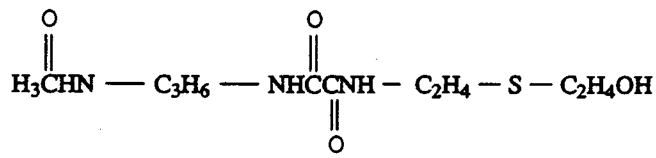 |
| 7.3 | 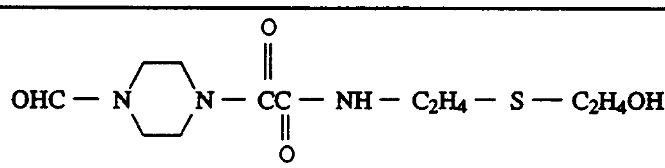 |
| 7.4 | 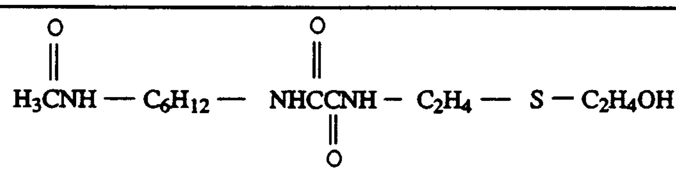 |

| Bsp.-Nr. | Formel |
|---|---|
| 7.5 | 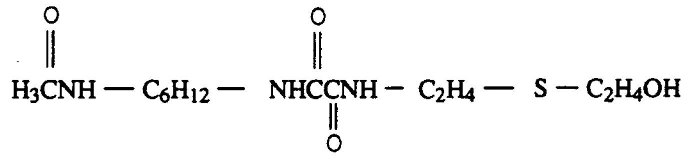 |
| 7.6 | 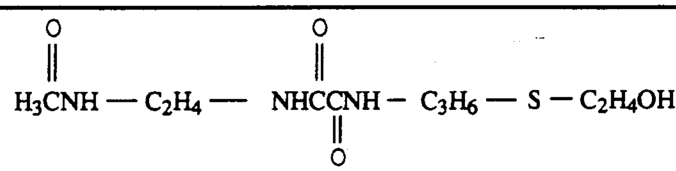 |
| 7.7 | 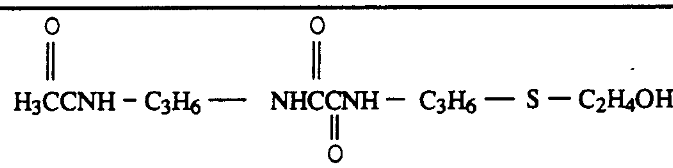 |
| 7.8 | 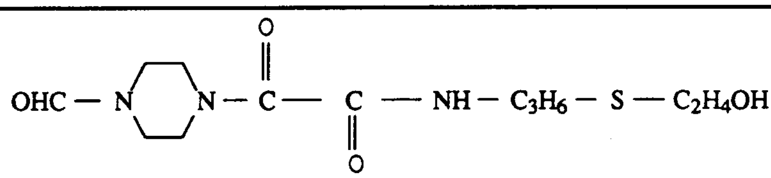 |
| 7.9 | 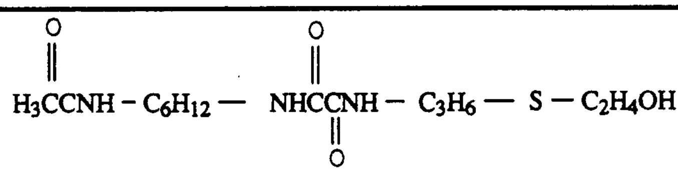 |

Beispiel 8.1

313 g (1 mol) der in Beispiel 4.1 beschriebenen Verbindung wurden in 2000 ml Wasser suspendiert, mit 2,8 g $Na_2WO_4$ x 2 $H_2O$ versetzt und auf 90 bis 95 °C erwärmt. Hierzu wurden 340 g (3 mol) 30 gew.-%iges wäßriges Wasserstoffperoxid so zugetropft, daß das Reaktionsgemisch schwach am Sieden gehalten werden konnte. Nach beendeter Zugabe wurde bei 90 bis 95 °C für 4 Stunden nachgerührt, anschließend abgekühlt, der entstandene Niederschlag abgetrennt und peroxidfrei gewaschen. Es entstanden 397 g einer Verbindung der Formel

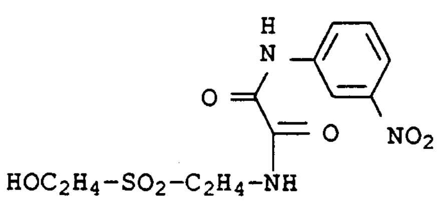

$^1$H-NMR ($d_6$-DMSO); $\delta$ = 3,37 (t,2H,$CH_2$); 3,52 (t,2H,$CH_2$); 3,77 (m,2H,$CH_2$); 3,95 (m,2H,$CH_2$); 5,10 (s,1H,OH); 7,68 (t,1H, Aromaten-H); 8,00 (d,1H, Aromaten-H); 8,25 (d,1H, Aromaten-H); 8,85 (s,1H, Aromaten-H); 9,07 (t,1H,NH); 11,07 (s,1H,NH) ppm.

In analoger Weise werden die in der folgenden Tabelle 8 aufgeführten Verbindungen erhalten.

Tabelle 8

| Bsp.-Nr. | Formel |
|---|---|
| 8.2 | 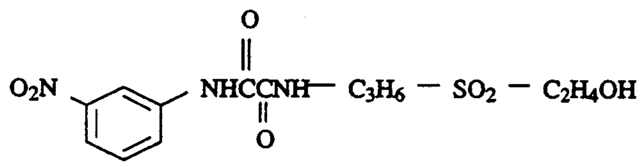 |
| 8.3 | 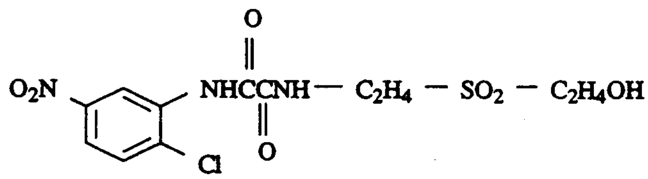 |
| 8.4 | 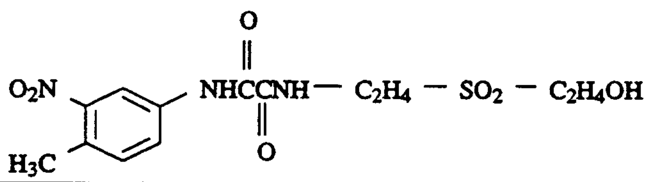 |
| 8.5 | 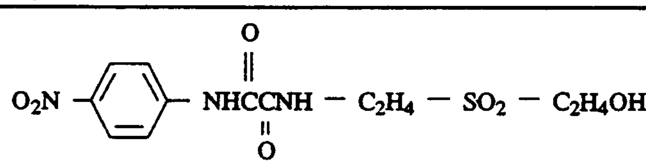 |

| Bsp.-Nr. | Formel |
|---|---|
| 8.6 | 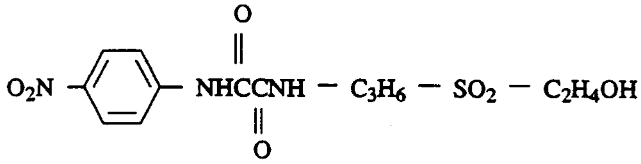 |
| 8.7 | 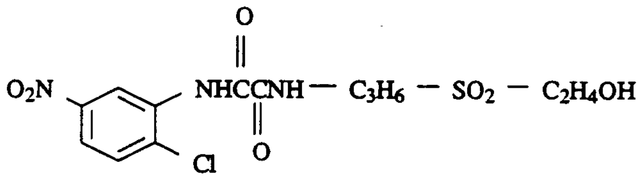 |
| 8.8 | 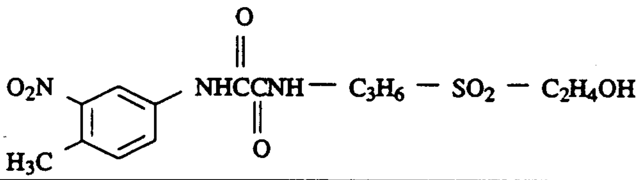 |
| 8.9 | 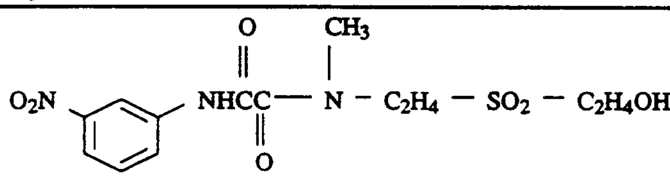 |
| 8.10 | 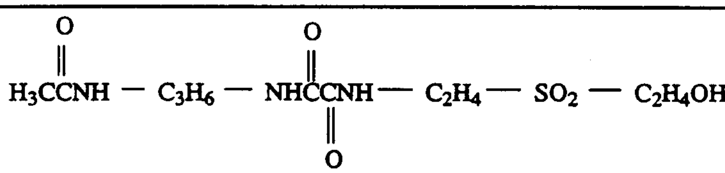 |
| 8.11 | 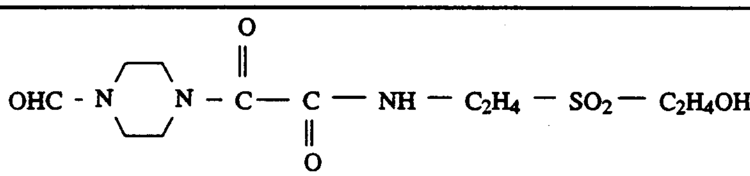 |
| 8.12 | 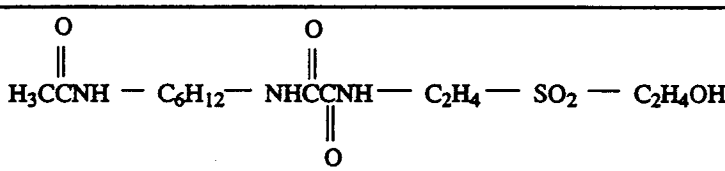 |
| 8.13 | 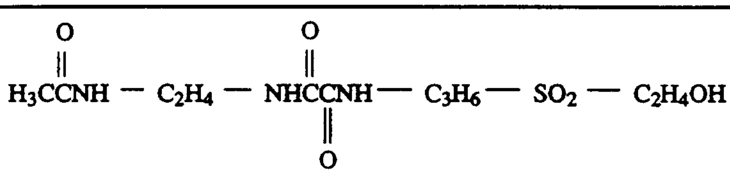 |

| Bsp.-Nr. | Formel |
|---|---|
| 8.14 | 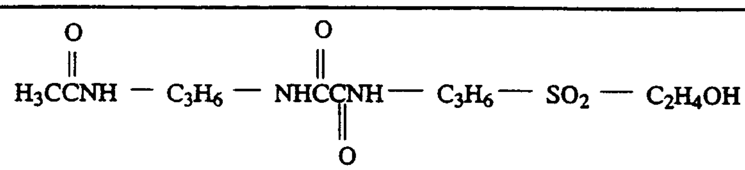 |
| 8.15 | 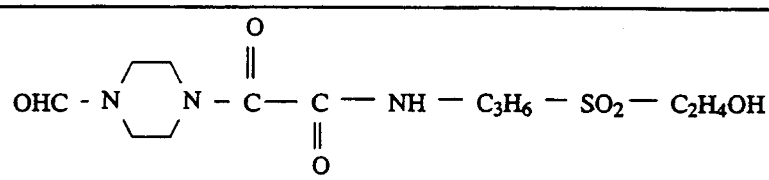 |
| 8.16 | 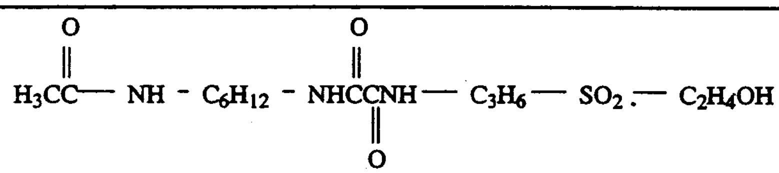 |
| 8.17 | 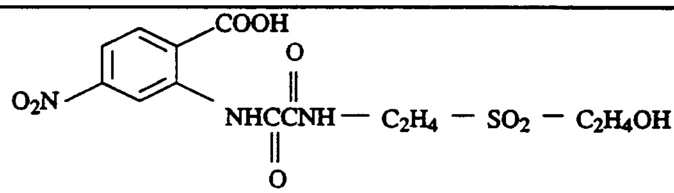 |
| 8.18 | 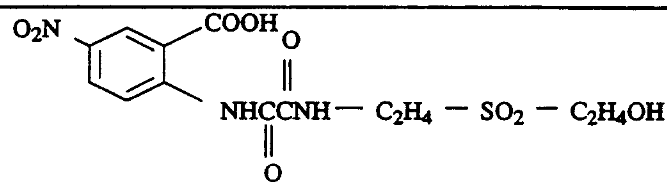 |
| 8.19 | 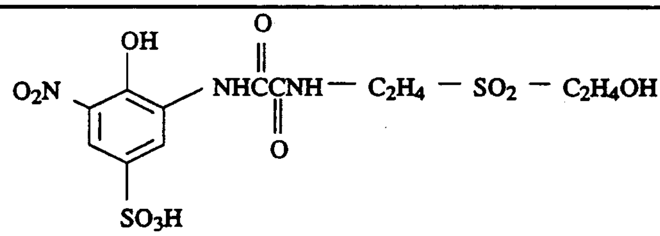 |
| 8.20 | 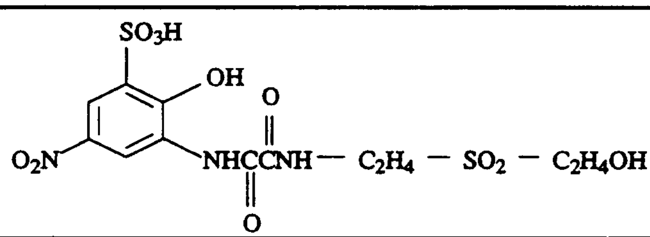 |

Beispiel 9.1

34,5 g (0,1 mol) der in Beispiel 8.1 beschriebenen Verbindung wurden in 100 ml Essigsäureanhydrid eingetragen und 1 Stunde auf 90 bis 95 °C erwärmt. Nach beendeter Umsetzung (DC-Kontrolle) wurde das überschüssige Anhydrid vorsichtig mit 100 ml Wasser zerstört, abgekühlt und der entstandene Niederschlag bei Raumtemperatur abgesaugt. Es verblieben 36,8 g einer Verbindung der Formel

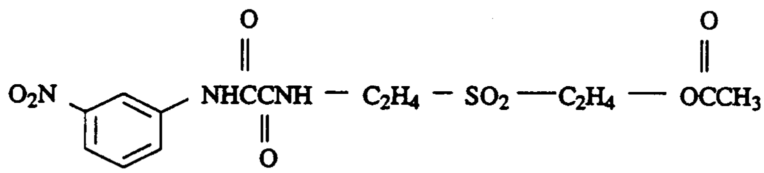

$^1$H-NMR ($d_6$-DMSO); δ = 2,07 (s,3H,$CH_3$); 3,45 (t,2H,$CH_2$); 3,60 (t,2H,$CH_2$); 3,70 (m,2H,$CH_2$); 4,37 (t,2H,$CH_2$); 7,67 (t,1H, Aromaten-H); 8,00 (d, 1H, Aromaten-H); 8,25 (d, 1H, Aromaten-H); 8,86 (t,1H, Aromaten-H); 9,27 (t,1H,NH); 11,85 (s,1H,NH) ppm.

In analoger Weise werden die in der folgenden Tabelle 9 aufgeführten Verbindungen erhalten.

Tabelle 9

| Bsp.-Nr. | Formel |
|---|---|
| 9.2 | 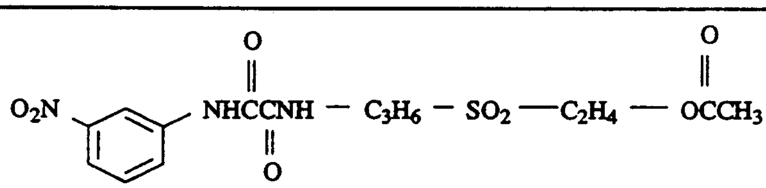 |
| 9.3 | 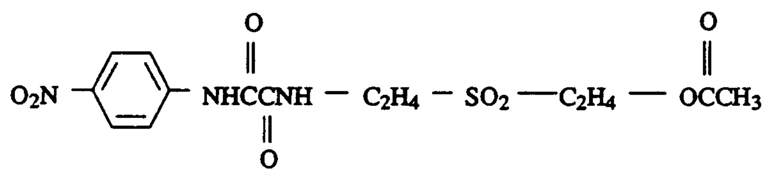 |

| Bsp.-Nr. | Formel |
|---|---|
| 9.4 | 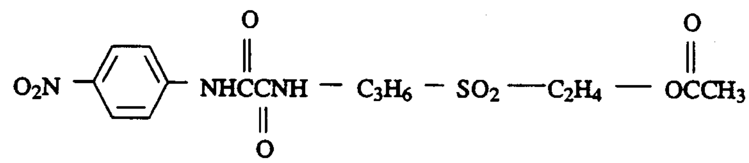 |
| 9.5 | 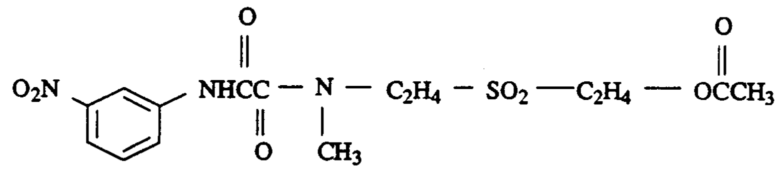 |
| 9.6 | 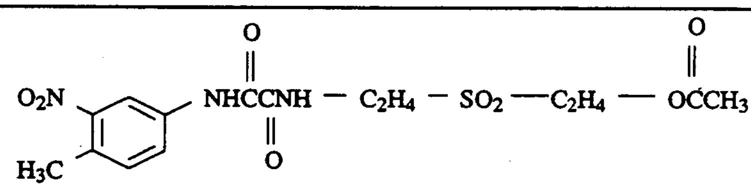 |
| 9.7 | 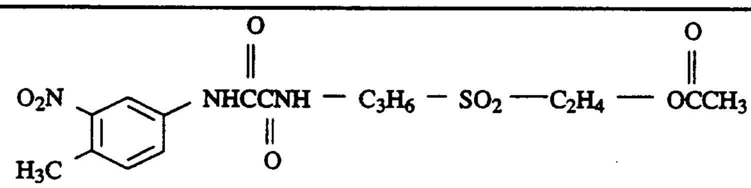 |
| 9.8 | 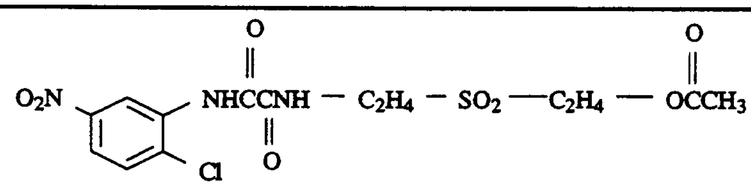 |
| 9.9 | 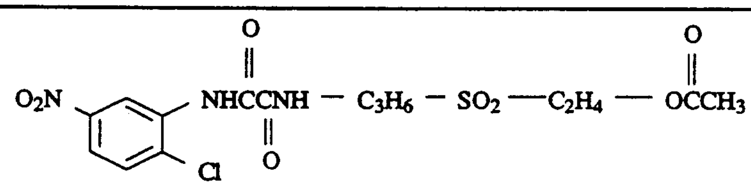 |
| 9.10 | 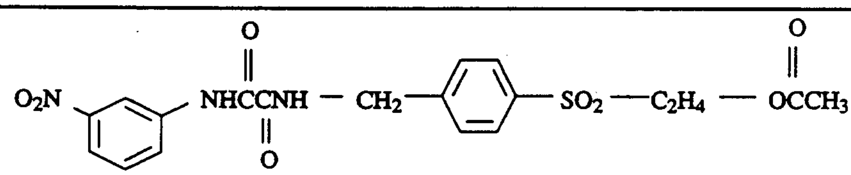 |
| 9.11 | 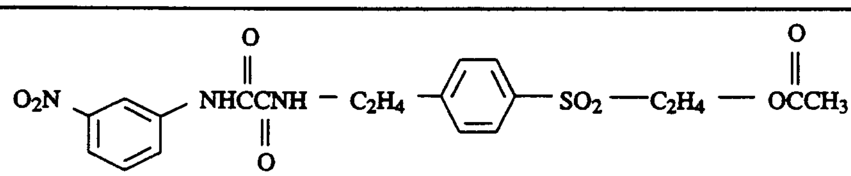 |

| Bsp.-Nr. | Formel |
|---|---|
| 9.12 | 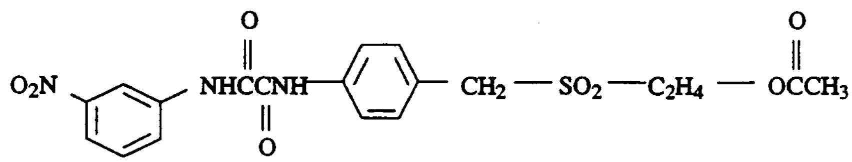 |
| 9.13 | 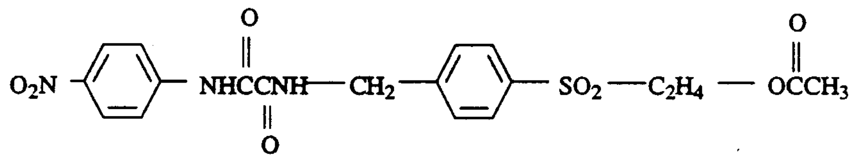 |
| 9.14 | 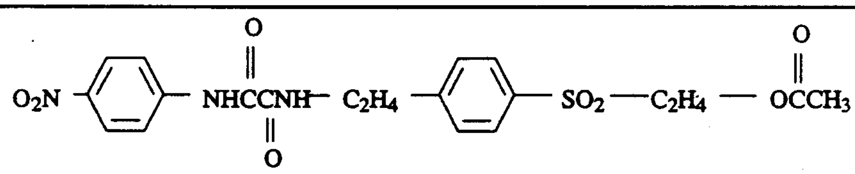 |
| 9.15 | 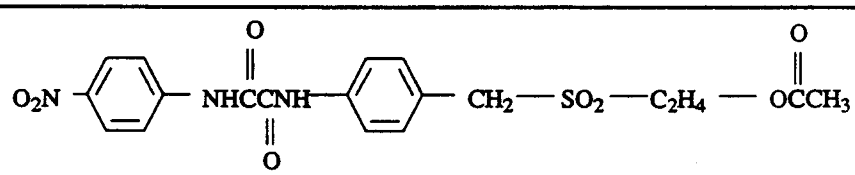 |

Beispiel 10.1

36,6 g (0,095 mol) der in Beispiel 9.1 beschriebenen Verbindung wurden mit 300 ml 70 gew.-%igem Ethanol und 1 g Palladium auf Kohle (10 Gew.-%) versetzt und bei 40 bis 50 °C hydriert. Nach beendeter Wasserstoffaufnahme wurde das Reaktionsgemisch zum Sieden erhitzt, vom Katalysator heiß abfiltriert und das Filtrat auf ca. 15 °C abgekühlt. Der entstandene Niederschlag wurde abgetrennt und getrocknet. Es verblieben 30,4 g einer Verbindung der Formel

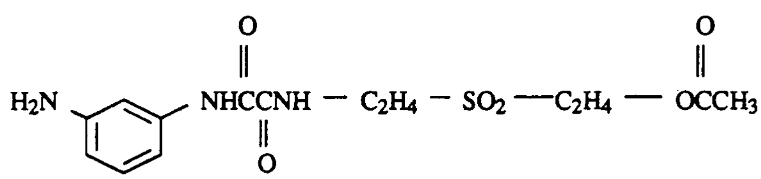

$^1$H-NMR ($d_6$-DMSO): $\delta$ = 2,08 (s,3H,$CH_3$), 3,05 (t,2H,$CH_2$); 3,58 (t,2H,$CH_2$); 3,75 (m,2H,$CH_2$); 4,41 (t,2H,$CH_2$); 5,07 (s,2H,$NH_2$); 6,45 (d,1H, Aromaten-H); 6,95-7,07 (m,2H, Aromaten-H); 7,16 (s,1H, Aromaten-H); 9,12 (t,1H,NH); 10,22 (s,1H,NH) ppm.

In analoger Weise werden die in der folgenden Tabelle 10 aufgeführten Verbindungen erhalten.

Tabelle 10

| Bsp.-Nr. | Formel |
|---|---|
| 10.2 | 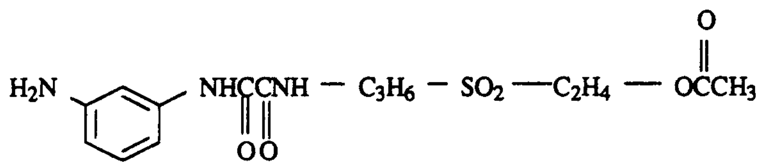 |
| 10.3 | 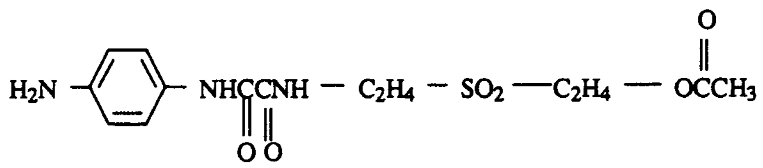 |

| Bsp.-Nr. | Formel |
|---|---|
| 10.4 | 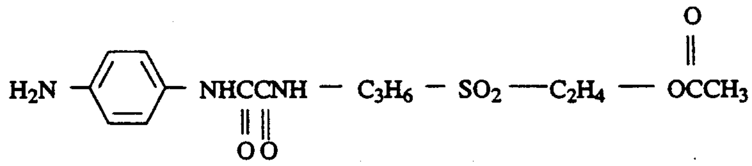 |
| 10.5 | 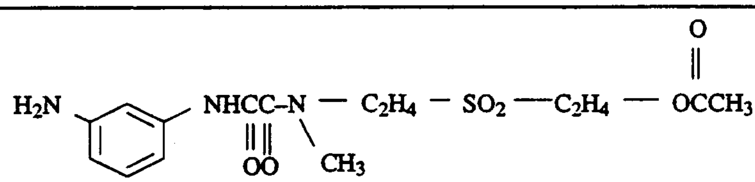 |
| 10.6 | 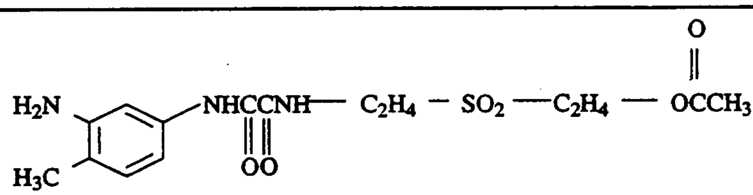 |
| 10.7 | 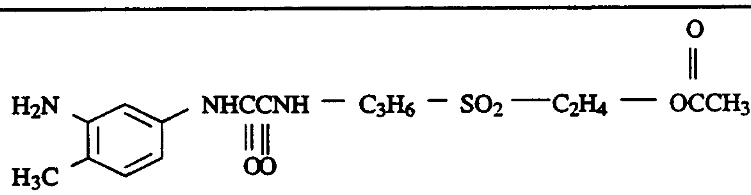 |
| 10.8 | 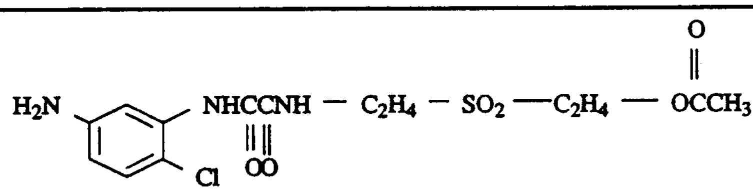 |
| 10.9 | 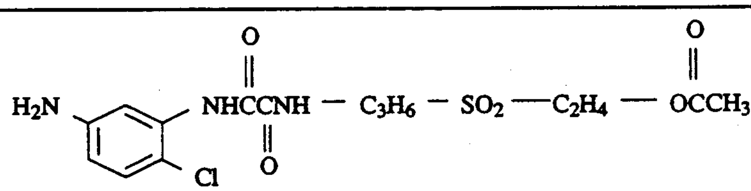 |
| 10.10 | 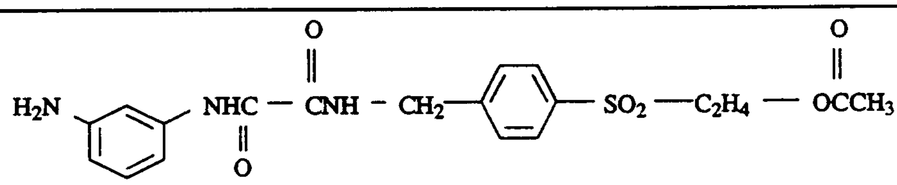 |
| 10.11 | 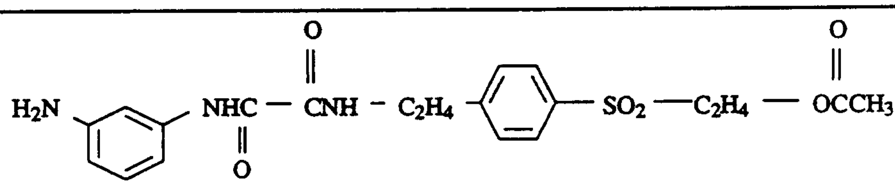 |

| Bsp.-Nr. | Formel |
|---|---|
| 10.12 | 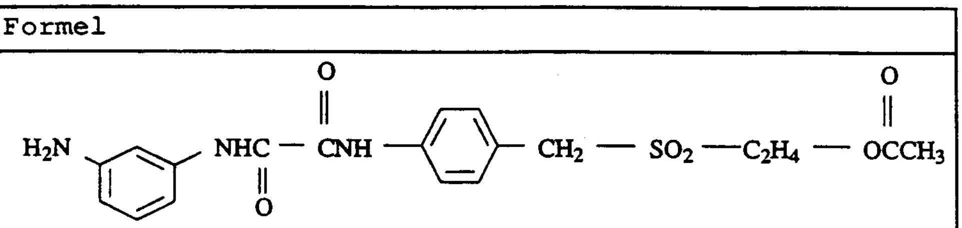 |
| 11.13 | 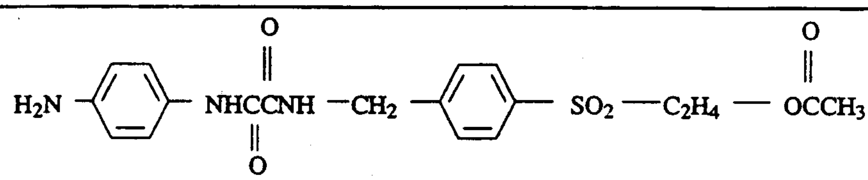 |
| 11.14 | 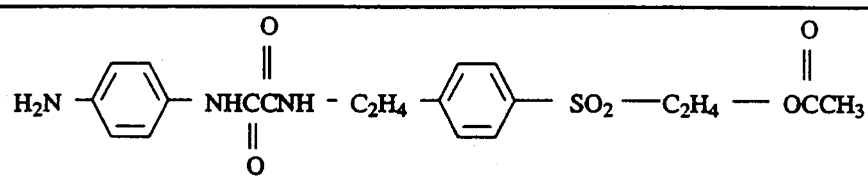 |
| 11.15 | 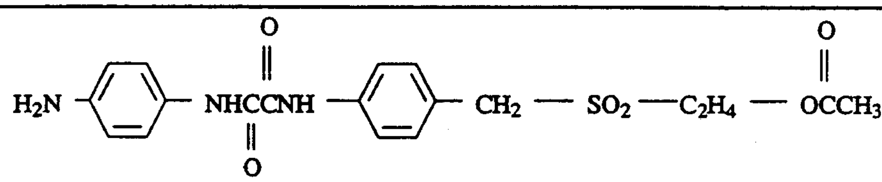 |

Beispiel 11.1

345 g (1 mol) der in Beispiel 8.1 beschriebenen Verbindung wurden in 1000 ml Methanol suspendiert, mit 15 g Raney-Nickel und 5 ml Propionsäure versetzt und bei 50 °C und 1 bis 3 bar Wasserstoffdruck hydriert. Nach beendeter Wasserstoffaufnahme wurde vom Katalysator abfiltriert, das Lösungsmittel weitgehend abgezogen und der verbleibende Rückstand isoliert. Es resultierten 268 g einer Verbindung der Formel

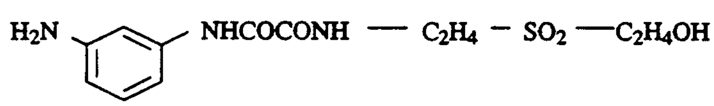

$^1$H-NMR ($d_6$-DMSO): $\delta$ = 3,33 (t, 2H, $CH_2$); 3,42 (t, 2H, $CH_2$); 3,65 (q, 2H, $CH_2$); 3,82 (q, 2H, $CH_2$); 5,17 (s, 2H, $NH_2$); 5,20 (t, 1H, OH); 6,33 (d, 1H, Aromaten-H); 6,85-7,00 (m, 2H, Aromaten-H); 7,10 (s, 1H, Aromaten-H); 9,07 (t, 1H, NH); 10,25 (s, 1H, NH) ppm.

In analoger Weise werden die in der folgenden Tabelle 11 aufgeführten Verbindungen erhalten.

Tabelle 11

| Bsp.-Nr. | Formel |
|---|---|
| 11.2 | 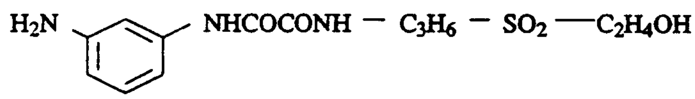 |
| 11.3 | 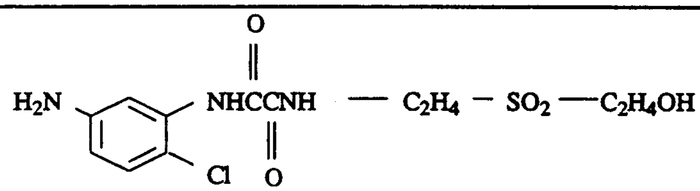 |
| 11,4 | 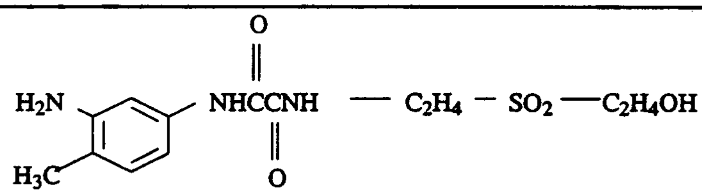 |
| 11.5 | 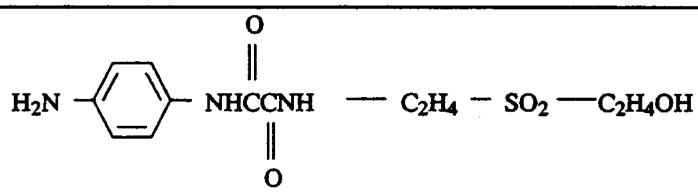 |
| 11.6 | 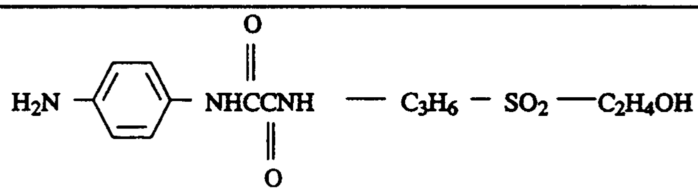 |
| 11.7 | 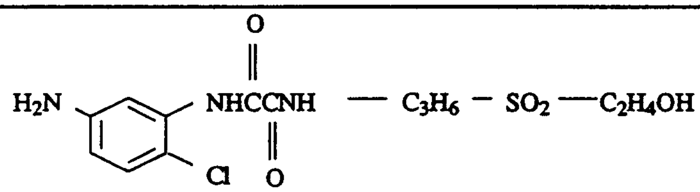 |

| Bsp.-Nr. | Formel |
|---|---|
| 11.8 | 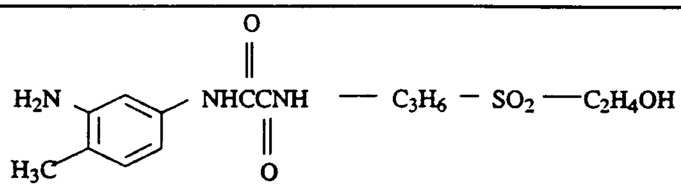 |
| 11.9 | 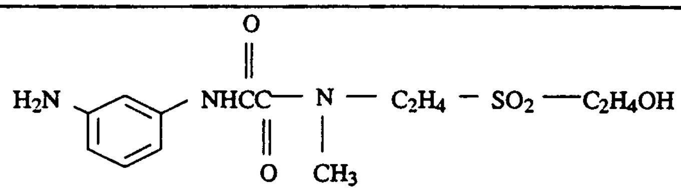 |
| 11.10 | 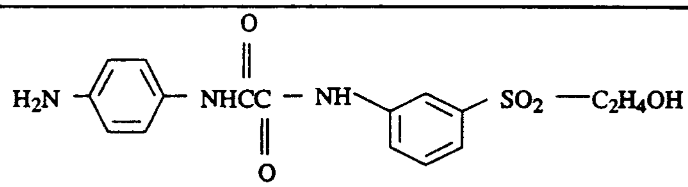 |
| 11.11 | 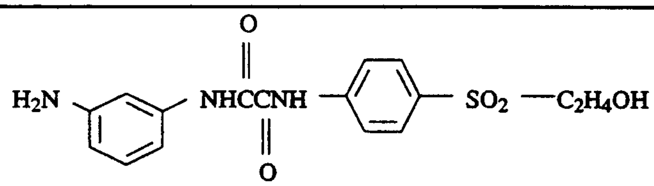 |
| 11.12 | 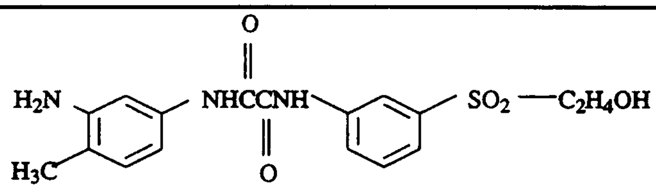 |
| 11.13 | 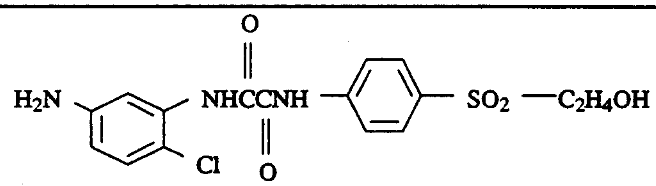 |
| 11.14 | 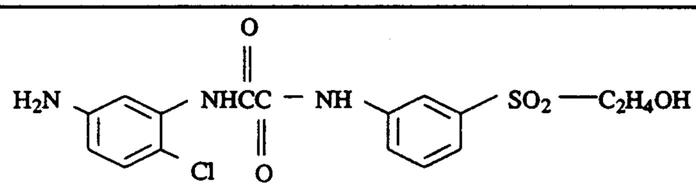 |
| 11.15 | 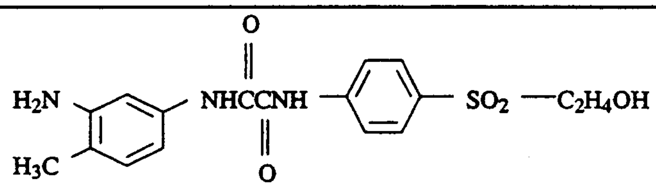 |

| Bsp.-Nr. | Formel |
| --- | --- |
| 11.16 | 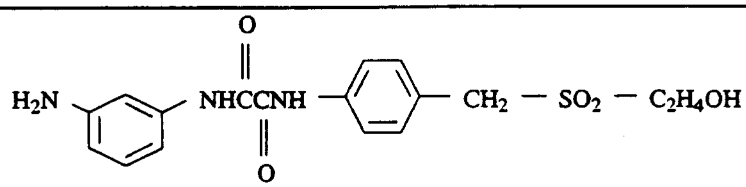 |
| 11.17 | 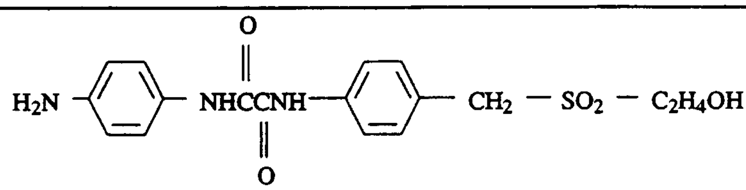 |
| 11.18 | 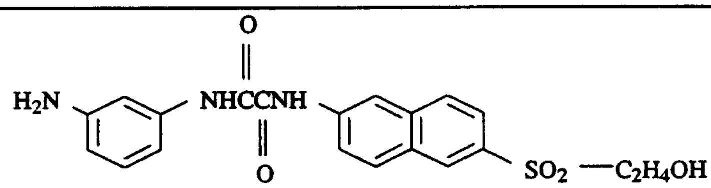 |
| 11.19 | 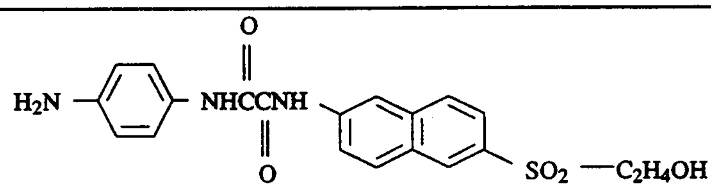 |
| 11.20 | 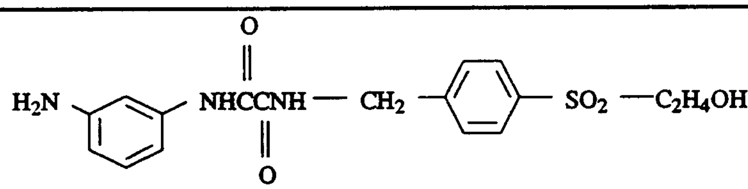 |
| 11.21 | 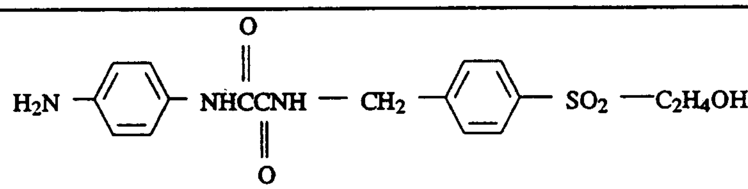 |
| 11.22 | 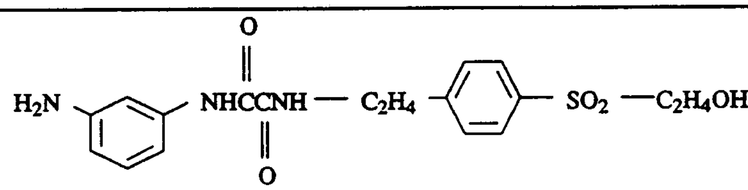 |
| 11.23 | 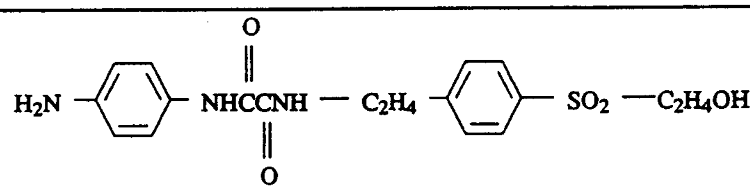 |

| Bsp.-Nr. | Formel |
|---|---|
| 11.24 | 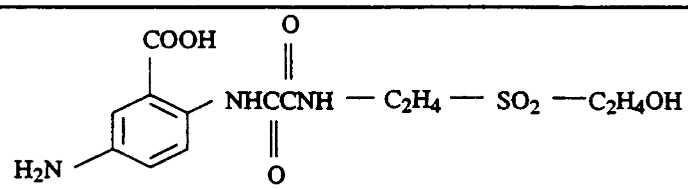 |
| 11.25 | 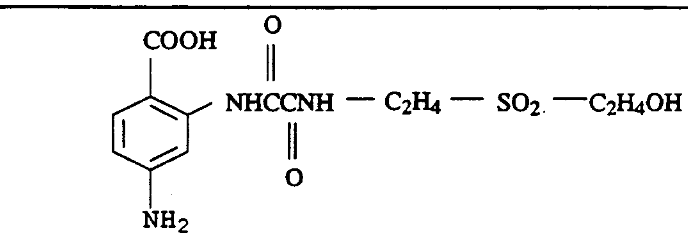 |
| 11.26 | 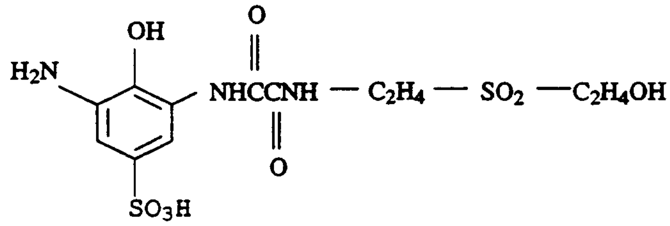 |
| 11.27 | 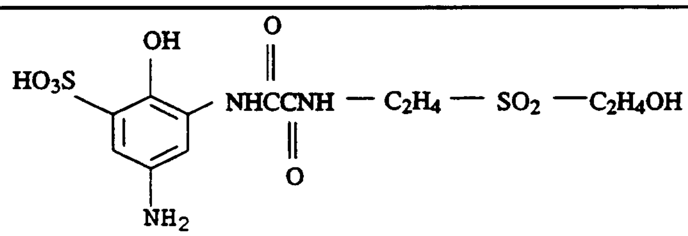 |
| 11.28 | 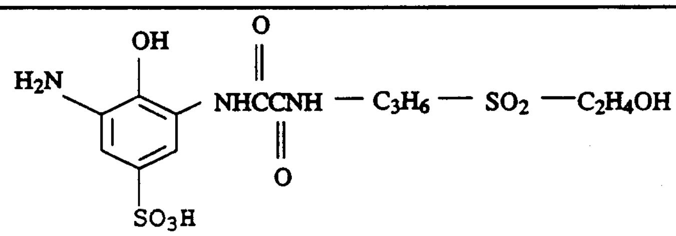 |
| 11.29 | 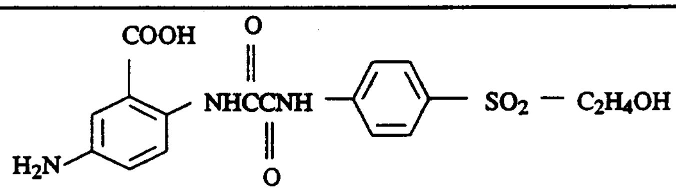 |
| 11.30 | 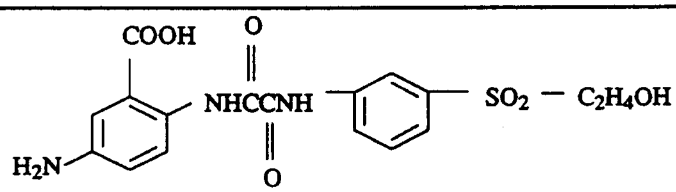 |

| Bsp.-Nr. | Formel |
|---|---|
| 11.31 | 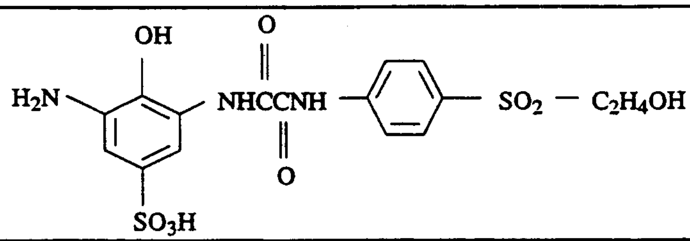 |
| 11.32 | 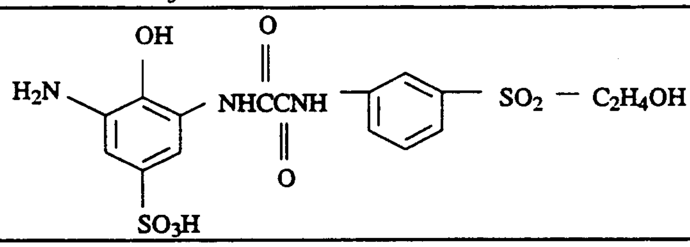 |

Beispiel 12.1

357 g (1 mol) der in Beispiel 5.1 beschriebenen Verbindung wurden in 500 ml 10 gew.-%iger Salzsäure 5 Stunden zum Sieden erhitzt. Anschließend wurde das Reaktionsgemisch weitgehend eingeengt, der verbleibende Rückstand abgesaugt und getrocknet. Es verblieben 236 g einer Verbindung der Formel

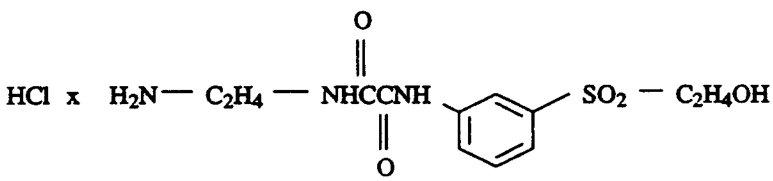

$^1$H-NMR ($d_6$-DMSO): $\delta$ = 3,01 (t, 2H, $CH_2$); 3,40-3,53 (m, 4H, $CH_2$); 3,70 (t, 2H, $CH_2$); 7,83-8,12 (m, 4H, Aromaten-H); 8,25 (s, 3H, $NH_3^+$); 9,18 (t, 1H, NH); 11,10 (s, 1H, NH) ppm.

In analoger Weise werden die in der folgenden Tabelle 12 aufgeführten Verbindungen erhalten.

Tabelle 12

| Bsp.-Nr. | Formel |
|---|---|
| 12.2 | 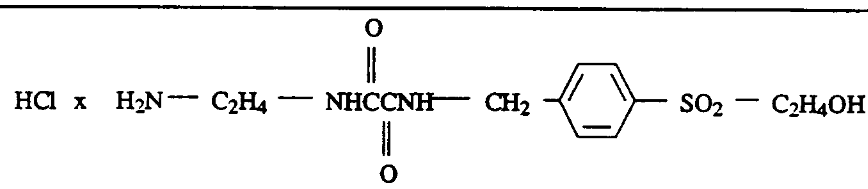 |
| 12.3 | 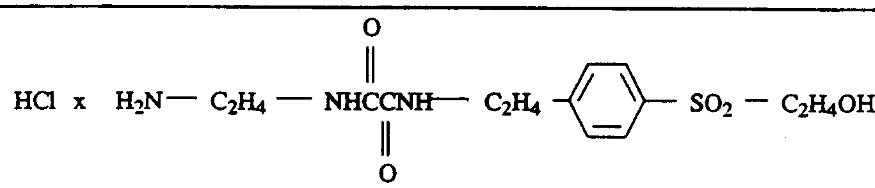 |
| 12.4 | 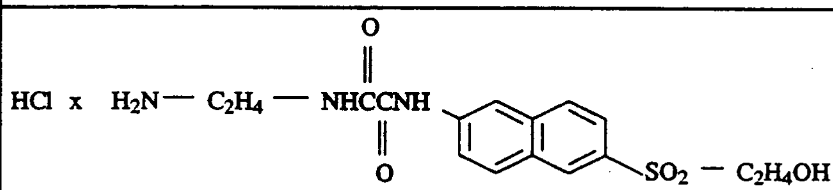 |
| 12.5 | 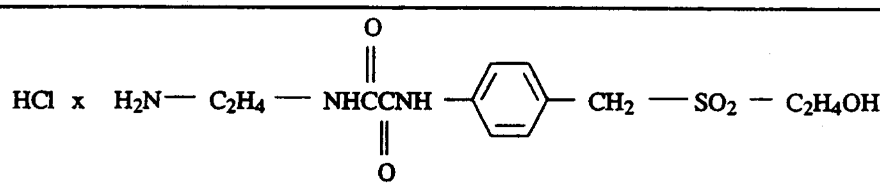 |
| 12.6 | 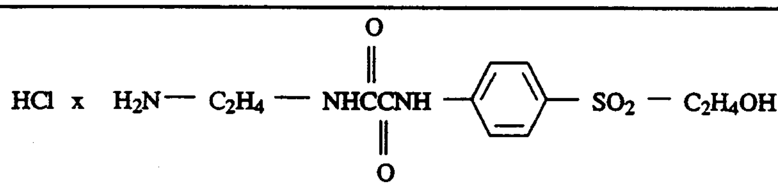 |
| 12.7 | 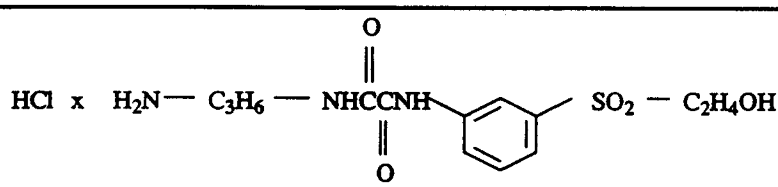 |
| 12.8 | 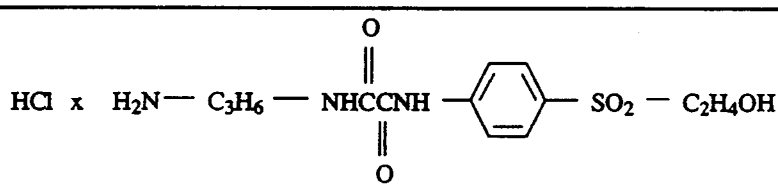 |

| Bsp.-Nr. | Formel |
|---|---|
| 12.9 | 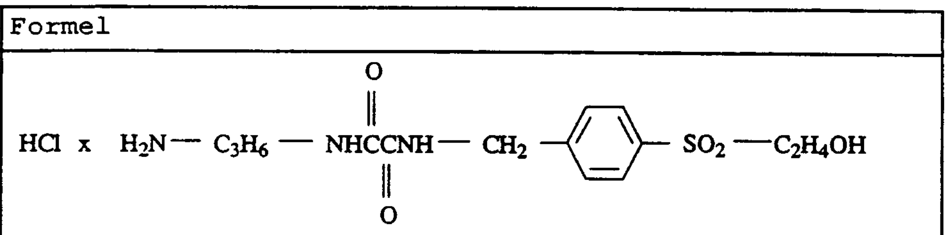 |
| 12.10 | 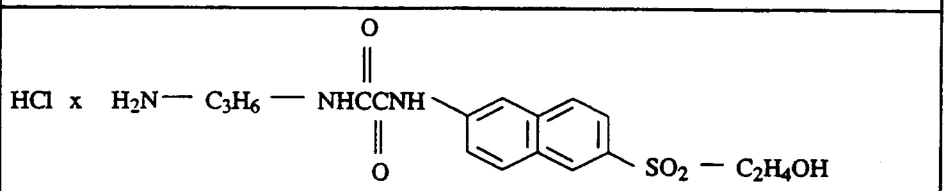 |
| 12.11 | 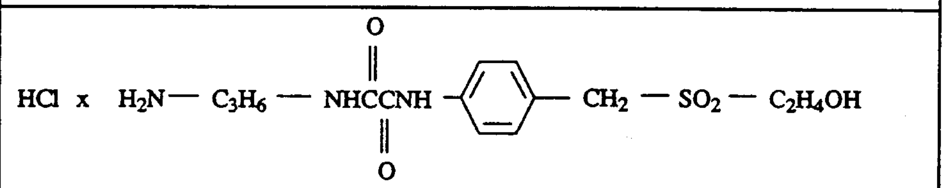 |
| 12.12 | 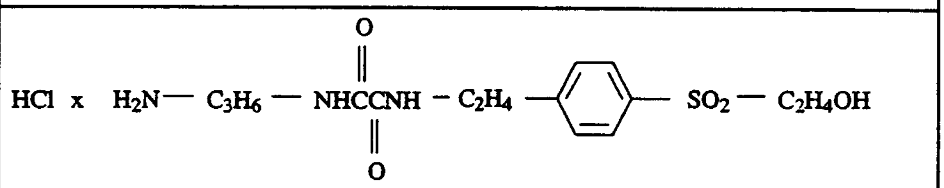 |
| 12.13 | 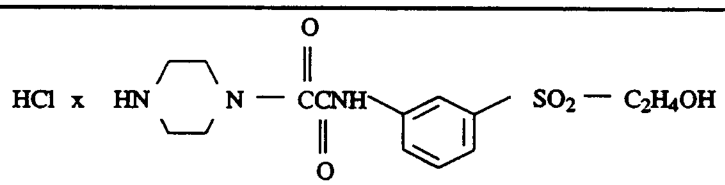 |
| 12.14 | 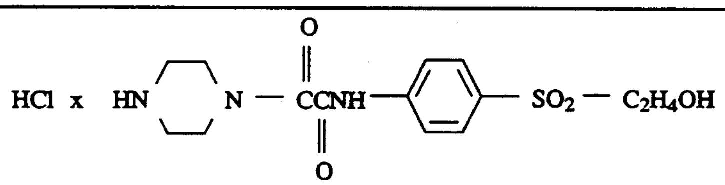 |
| 12.15 | 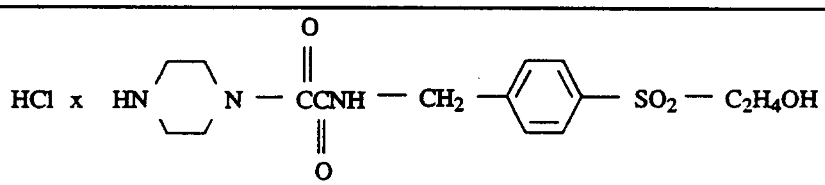 |
| 12.16 | 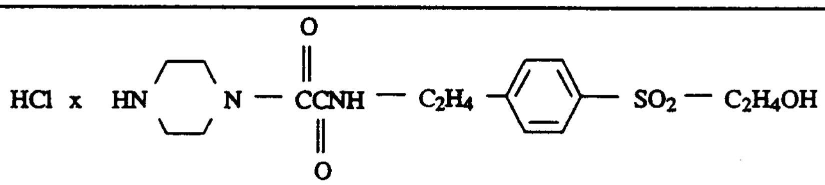 |

| Bsp.-Nr. | Formel |
|---|---|
| 12.17 | 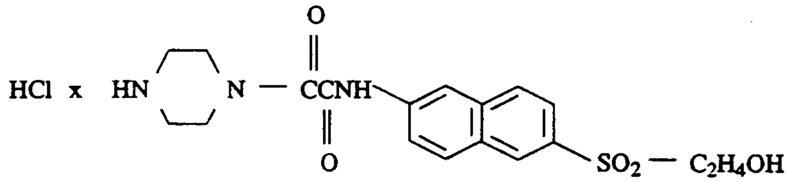 |
| 12.18 | 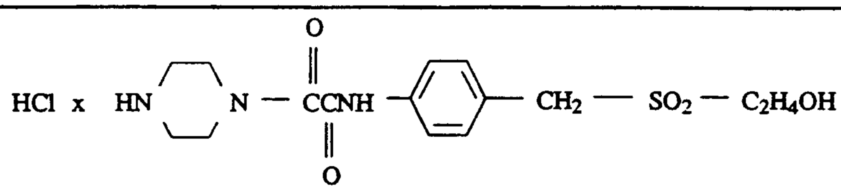 |

Beispiel 13.1

315 g (1 mol) der in Beispiel 11.1 beschriebenen Verbindung wurden bei 20 bis 30 °C in 680 ml konz. Schwefelsäure eingetragen und bis zur vollständigen Umsetzung (DC-Kontrolle) bei Raumtemperatur gerührt. Die schwefelsaure Lösung wurde auf 900 g Eis zerlegt, der entstandene Niederschlag abgesaugt und getrocknet. Es verblieben 350 g einer Verbindung der Formel

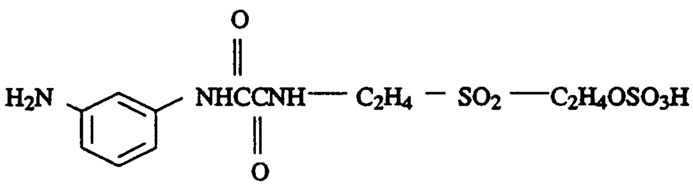

$^1$H-NMR ($d_6$-DMSO): $\delta$ = 3,40 (t, 2H, $CH_2$); 3,48 (t, 2H, $CH_2$); 3,70 (q, 2H $CH_2$); 4,17 (t, 2H, $CH_2$), 7,12 (d, 1H, Aromaten-H), 7,48 (t, 1H, Aromaten-H); 7,78 (d, 1H, Aromaten-H); 8,03 (s, 1H, Aromaten-H); 9,10 (t, 1H, NH); 9,50 (s, 3H, $NH_3^+$); 10,98 (s, 1H, NH) ppm.

In analoger Weise werden die in der folgenden Tabelle 13 aufgeführten Verbindungen erhalten, wobei gegebenenfalls ausgesalzen werden muß.

Tabelle 13

| Bsp.-Nr. | Formel |
|---|---|
| 13.2 | 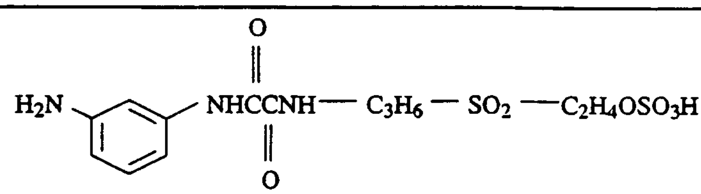 |
| 13.3 | 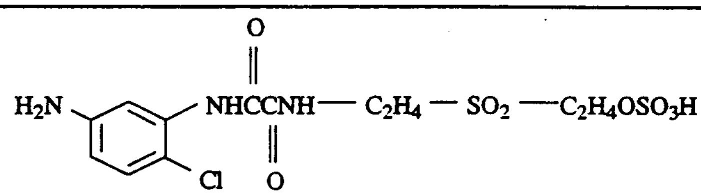 |
| 13.4 | 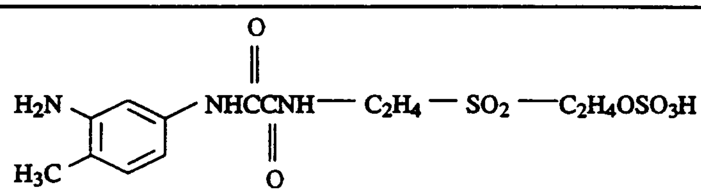 |
| 13.5 | 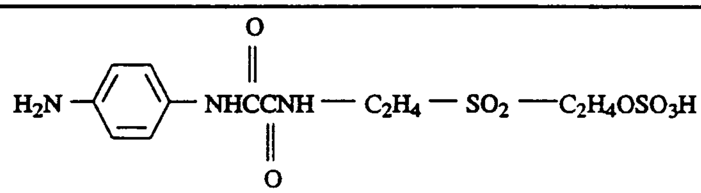 |
| 13.6 | 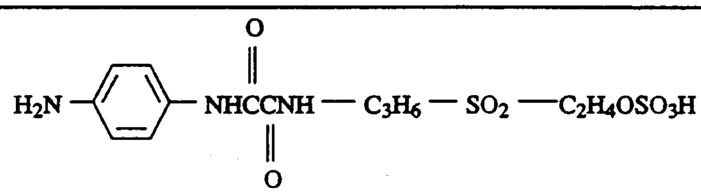 |
| 13.7 | 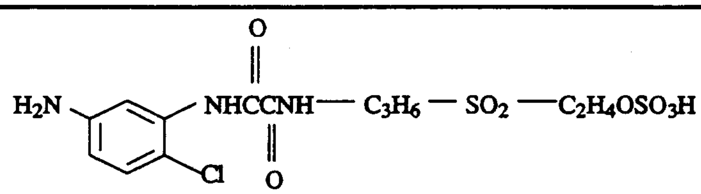 |
| 13.8 | 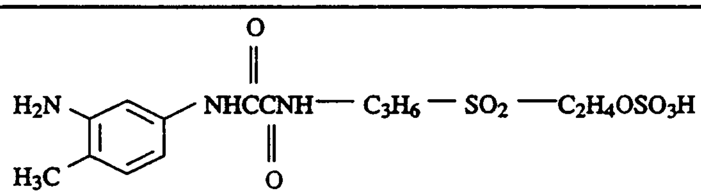 |

| Bsp.-Nr. | Formel |
|---|---|
| 13.9 | 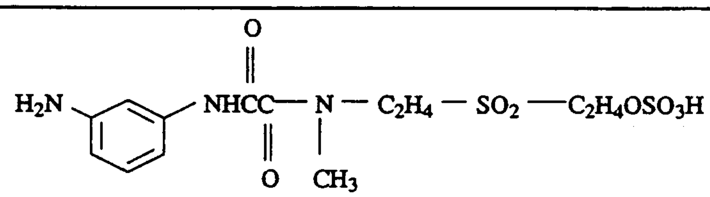 |
| 13.10 | 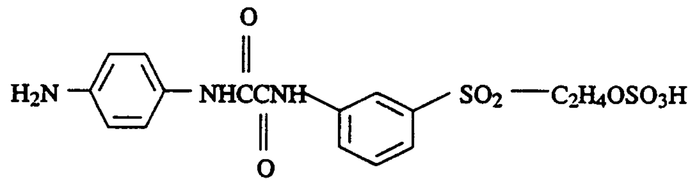 |
| 13.11 | 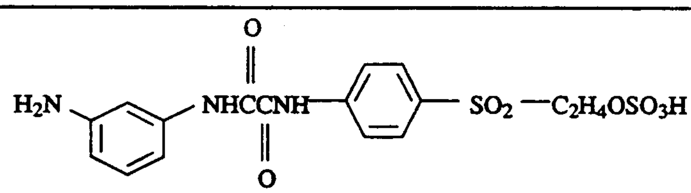 |
| 13.12 | 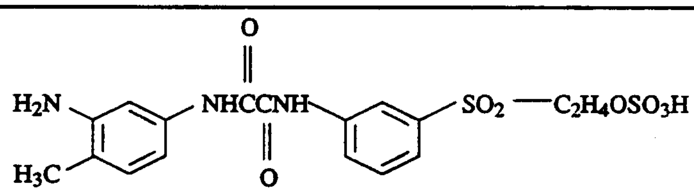 |
| 13.13 | 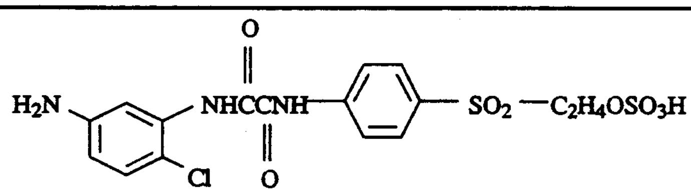 |
| 13.14 | 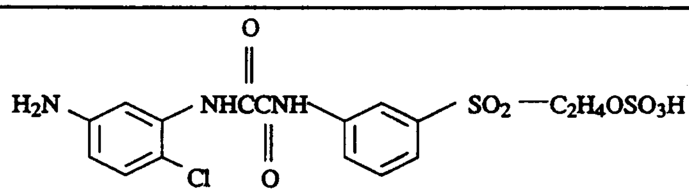 |
| 13.15 | 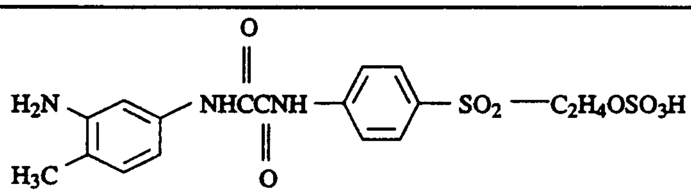 |
| 13.16 | 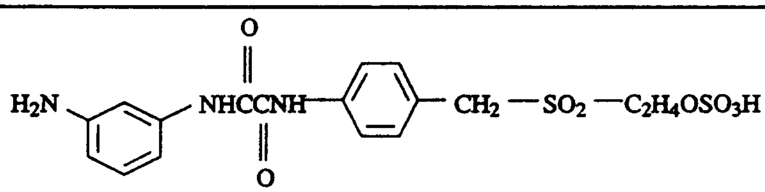 |

| Bsp.-Nr. | Formel |
|---|---|
| 13.17 | 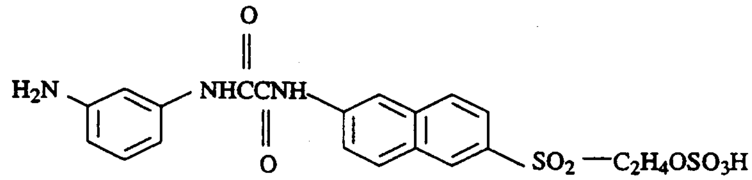 |
| 13.18 | 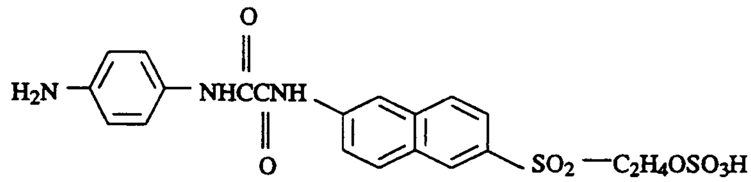 |
| 13.19 | 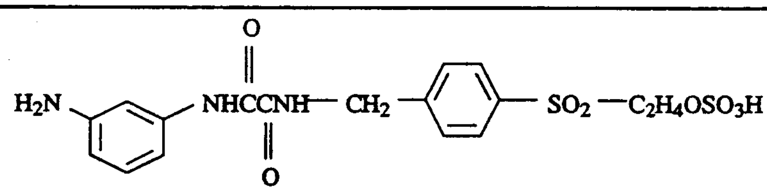 |
| 13.20 | 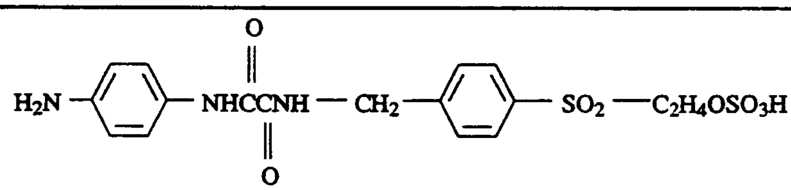 |
| 13.21 | 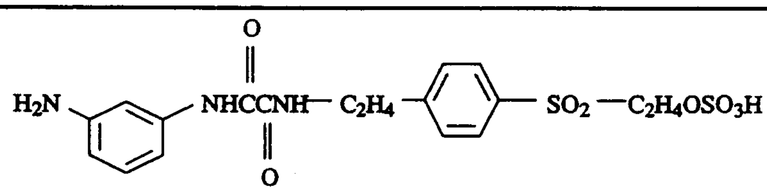 |
| 13.22 | 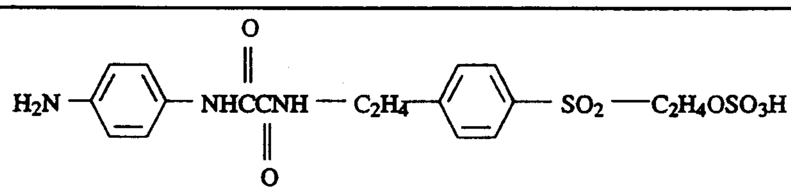 |
| 13.23 | 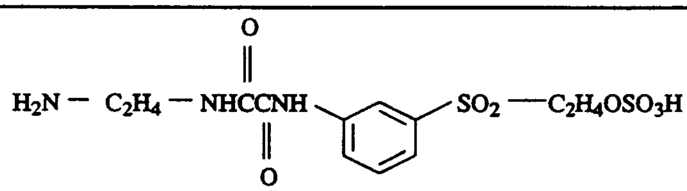 |
| 13.24 | 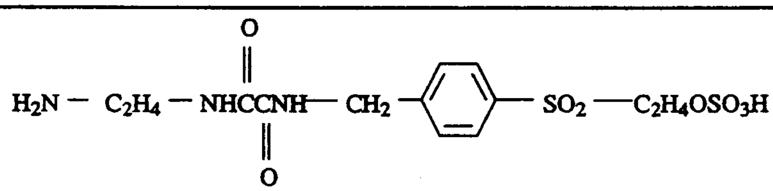 |

| Bsp.-Nr. | Formel |
|---|---|
| 13.25 | 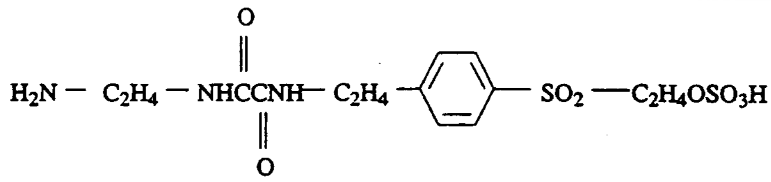 |
| 13.26 | 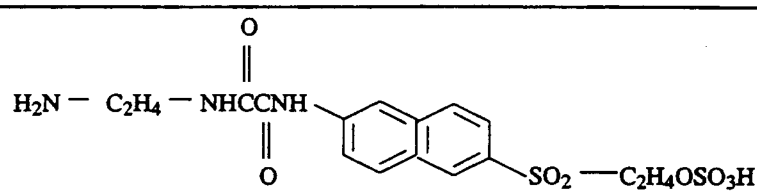 |
| 13.27 | 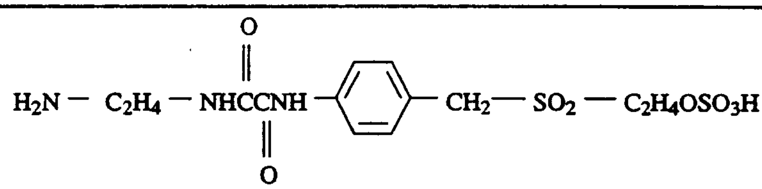 |
| 13.28 | 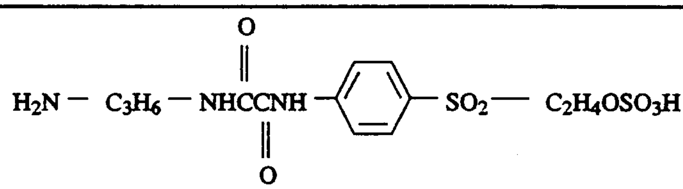 |
| 13.29 | 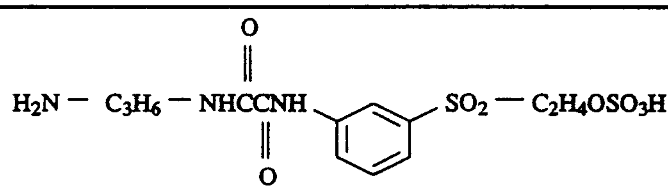 |
| 13.30 | 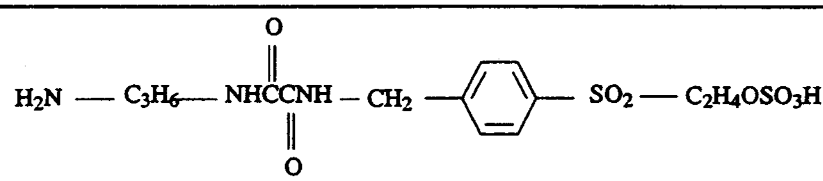 |
| 13.31 | 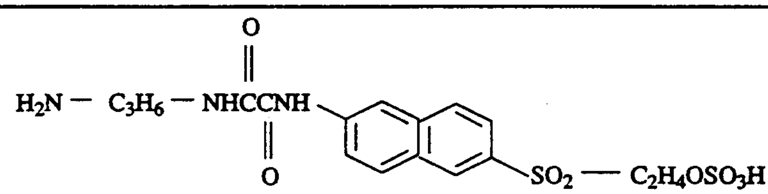 |
| 13.32 | 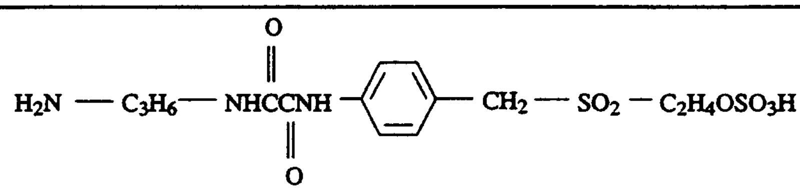 |

| Bsp.-Nr. | Formel |
|---|---|
| 13.33 | 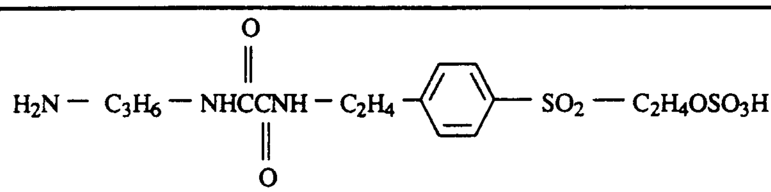 |
| 13.34 | 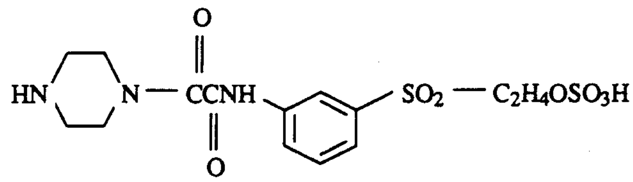 |
| 13.35 | 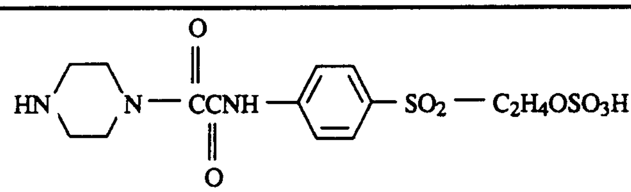 |
| 13.36 | 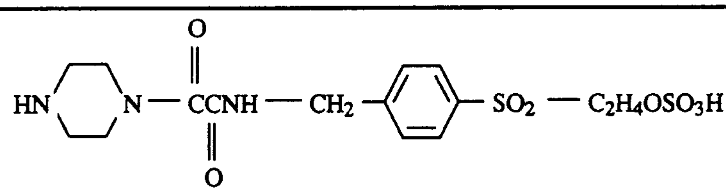 |
| 13.37 | 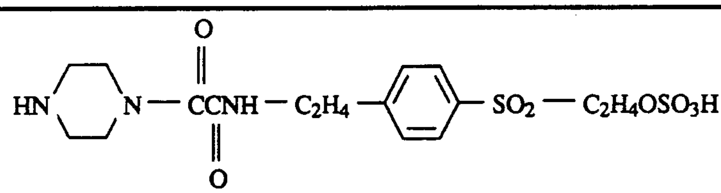 |
| 13.38 | 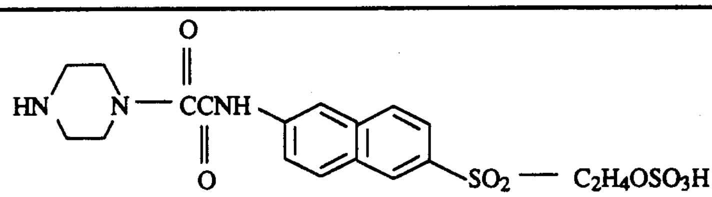 |
| 13.39 | 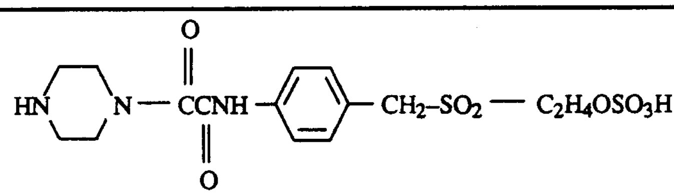 |
| 13.40 | 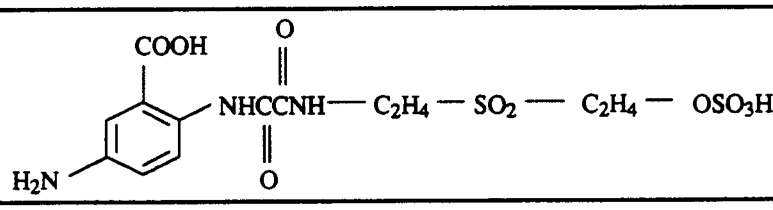 |

| Bsp.-Nr. | Formel |
| --- | --- |
| 13.41 | 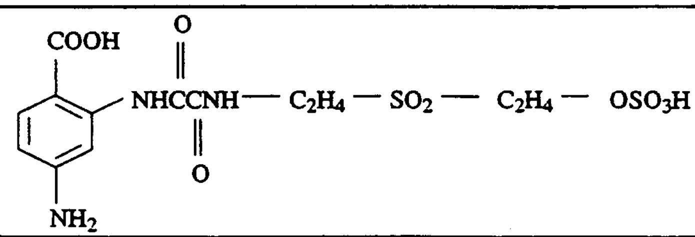 |
| 13.42 | 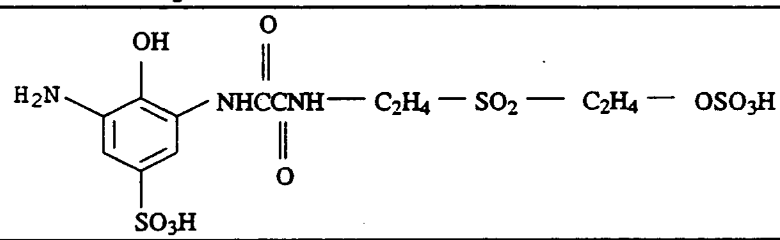 |
| 13.43 | 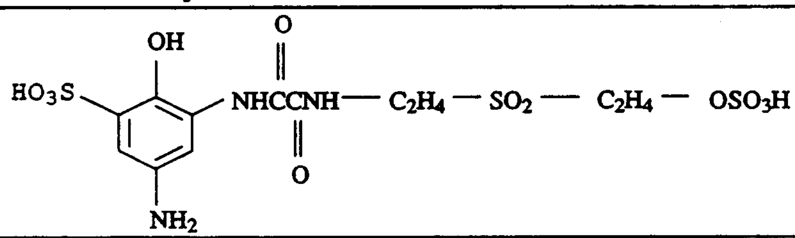 |
| 13.44 | 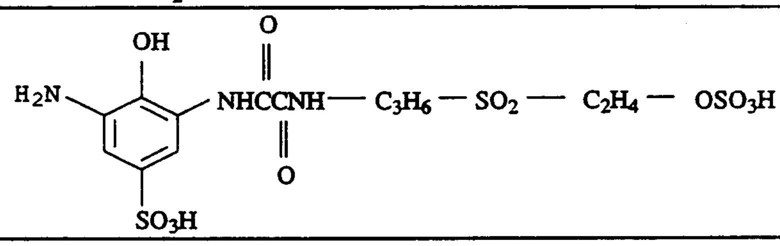 |
| 13.45 | 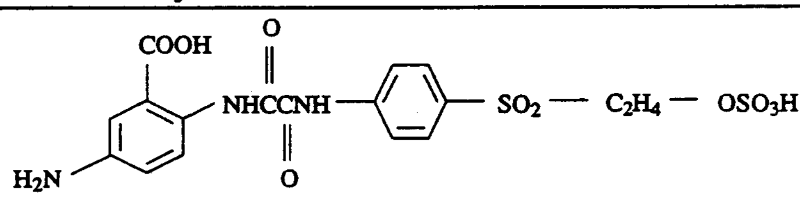 |
| 13.46 | 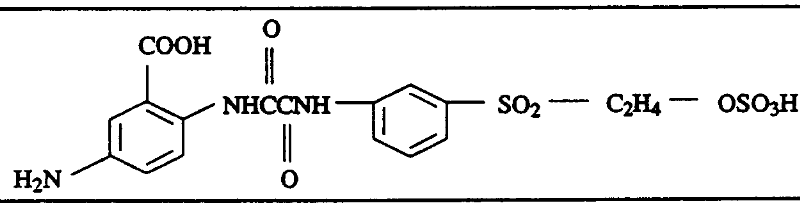 |

| Bsp.-Nr. | Formel |
|---|---|
| 13.47 | 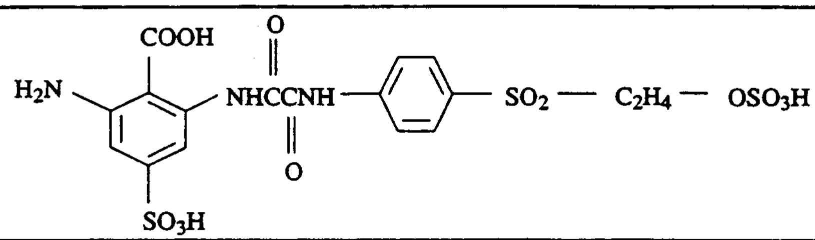 |
| 13.48 | 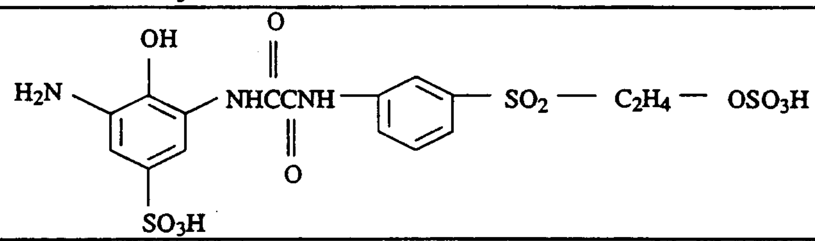 |

Beispiel 14.1

47 g (0,109 mol) der in Beispiel 13.3 beschriebenen Verbindung wurden in 80 ml Wasser suspendiert und mit 2N Natronlauge auf einen pH-Wert von 10 gestellt und bei Raumtemperatur bis zur vollständigen Eliminierung bei diesem pH-Wert gehalten. Anschließend wurde das Reaktionsgemisch mit 10 gew.-%iger Salzsäure auf einen pH-Wert von 5 gestellt, das Produkt abgesaugt und getrocknet. Es verblieben 34,7 g einer Verbindung der Formel

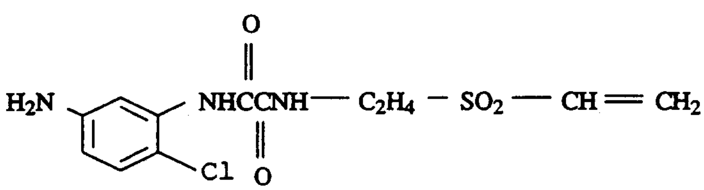

$^1$H-NMR ($d_6$-DMSO): $\delta$ = 3,38 (m, 2H, $CH_2$); 3,63 (m, 2H, $CH_2$); 5,50 (s, 2H, $NH_2$); 6,26-6,47 (m, 3H, $CH=CH_2$); 7.00-7,35 (m, 3H, Aromaten-H); 9,12 (t, 1H, NH); 9,87 (s, 1H, NH) ppm.

In analoger Weise werden die in Tabelle 14 aufgeführten Verbindungen erhalten.

Tabelle 14

| Bsp.-Nr. | Formel |
|---|---|
| 14.2 | 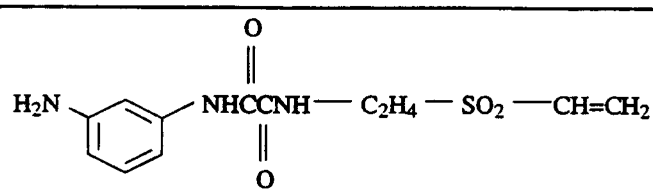 |
| 14.3 | 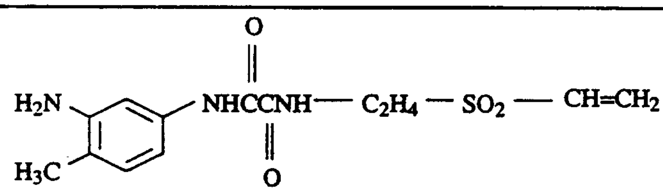 |
| 14.4 | 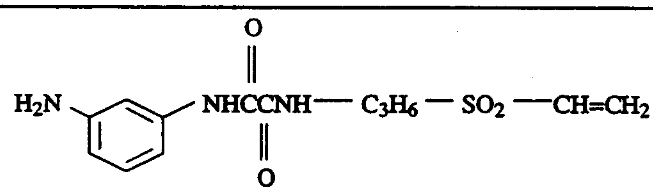 |
| 14.5 | 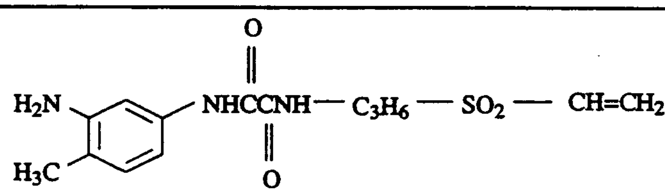 |
| 14.6 | 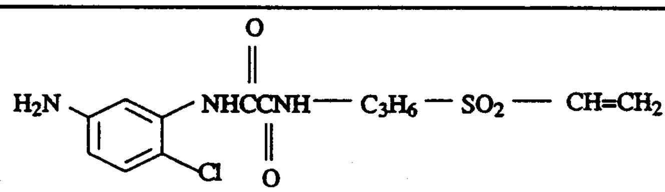 |
| 14.7 | 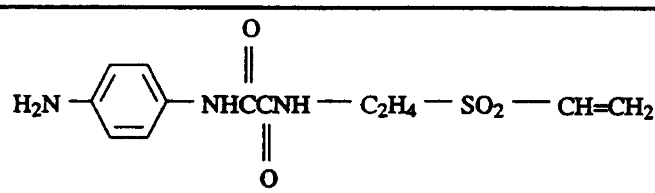 |
| 14.8 | 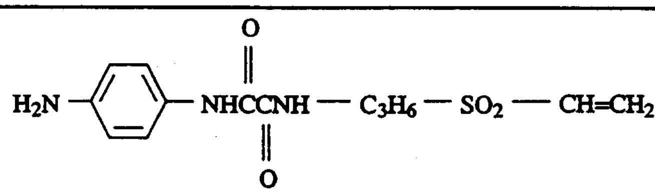 |

| Bsp.-Nr. | Formel |
|---|---|
| 14.9 | 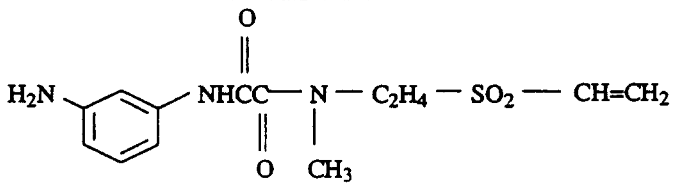 |
| 14.10 | 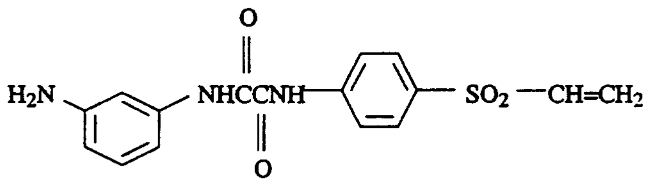 |
| 14.11 | 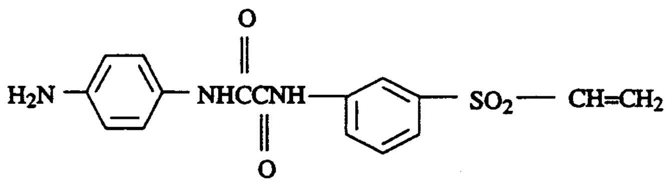 |
| 14.12 | 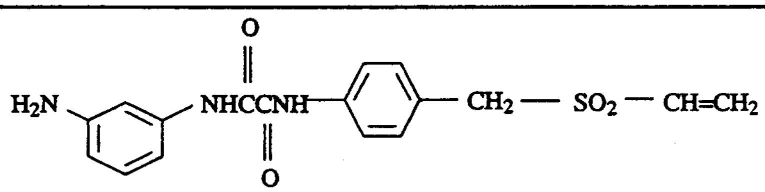 |
| 14.13 | 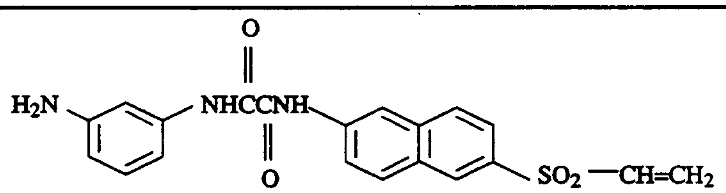 |
| 14.14 | 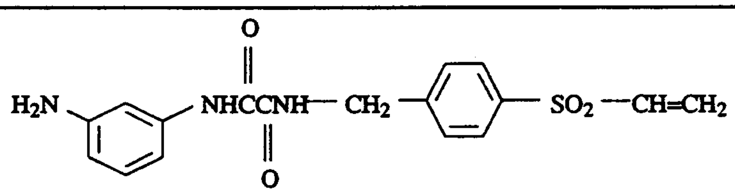 |
| 14.15 | 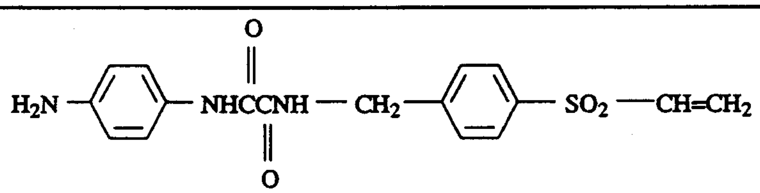 |
| 14.16 | 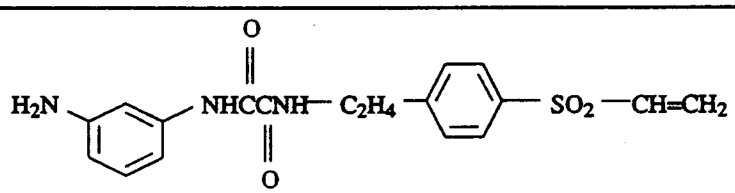 |

| Bsp.-Nr. | Formel |
|---|---|
| 14.17 | 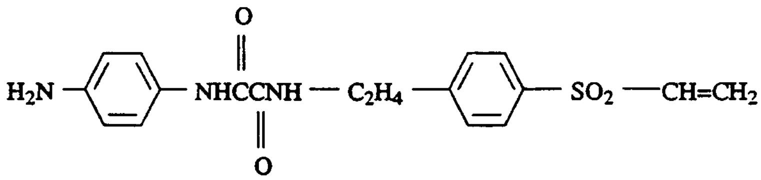 |
| 14.18 | 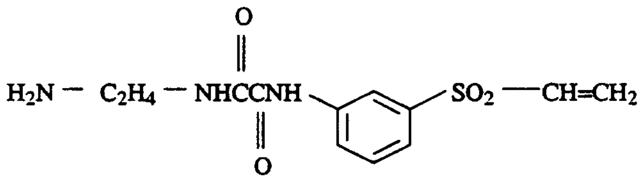 |
| 14.19 | 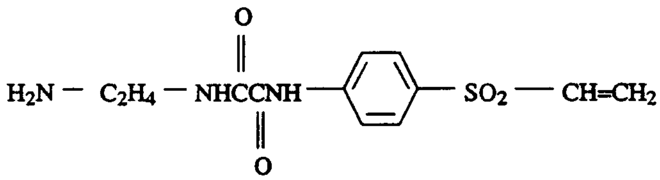 |
| 14.20 | 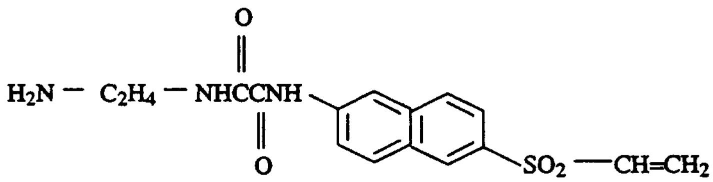 |
| 14.21 | 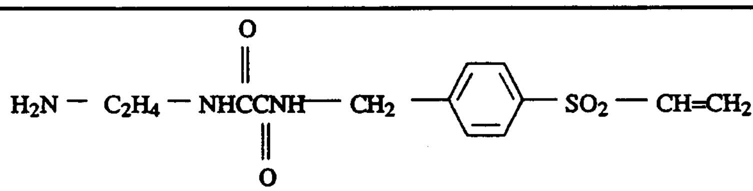 |
| 14.22 | 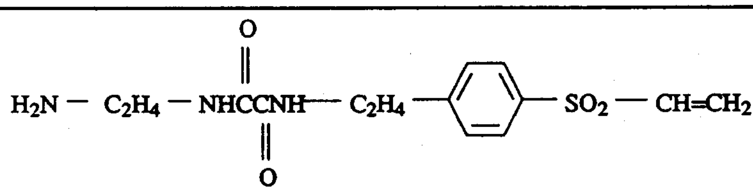 |
| 14.23 | 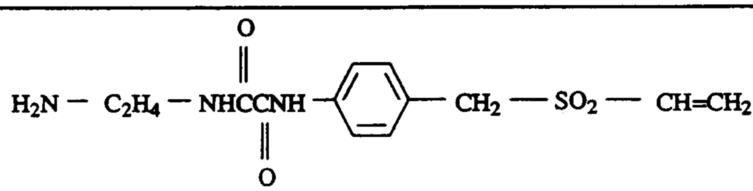 |
| 14.24 | 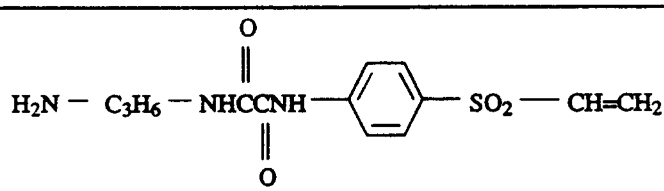 |

| Bsp.-Nr. | Formel |
|---|---|
| 14.25 | 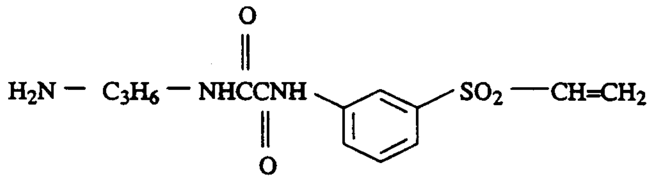 |
| 14.26 | 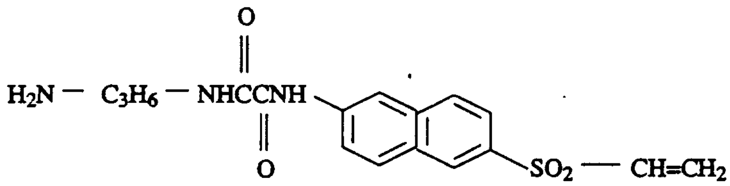 |
| 14.27 | 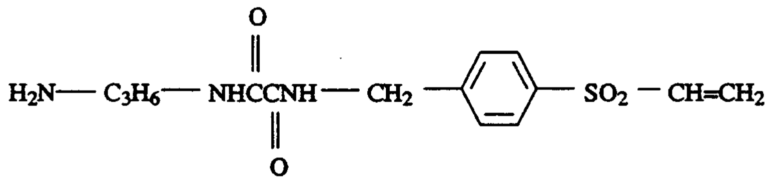 |
| 14.28 | 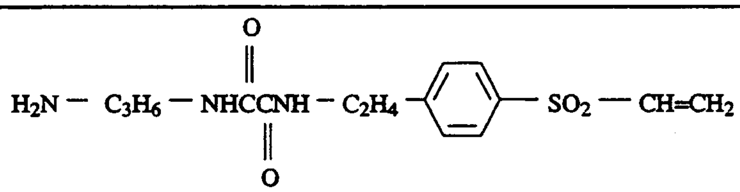 |
| 14.29 | 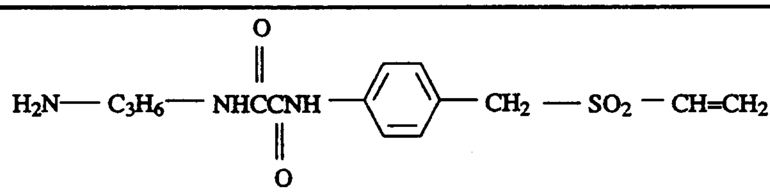 |
| 14.30 | 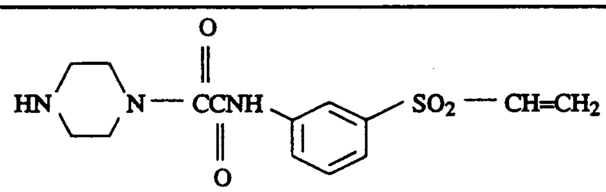 |
| 14.31 | 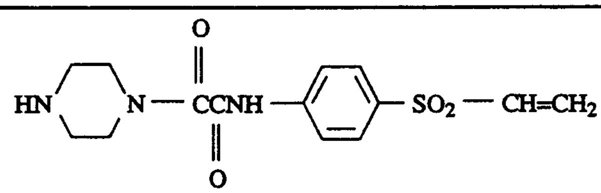 |
| 14.32 | 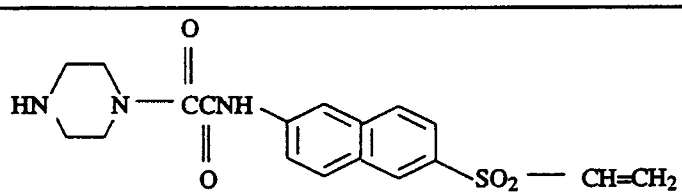 |

| Bsp.-Nr. | Formel |
|---|---|
| 14.33 | 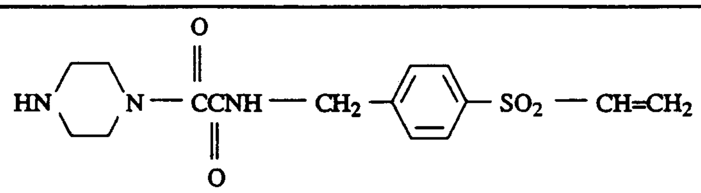 |
| 14.34 | 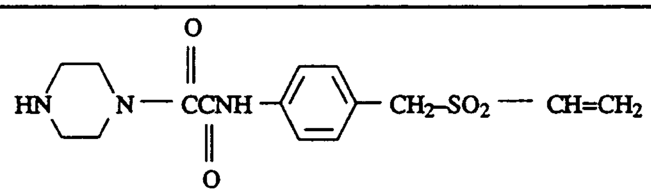 |
| 14.35 | 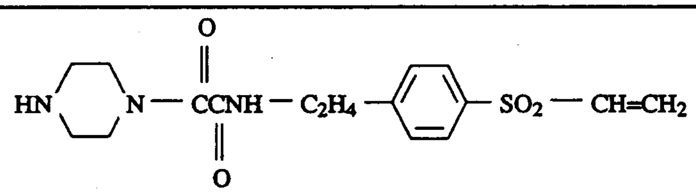 |
| 14.36 | 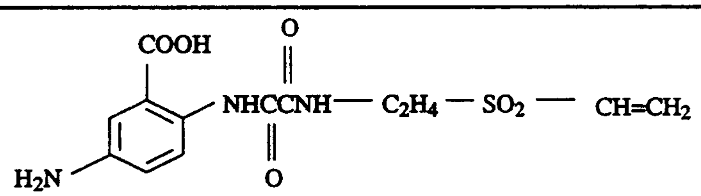 |
| 14.37 | 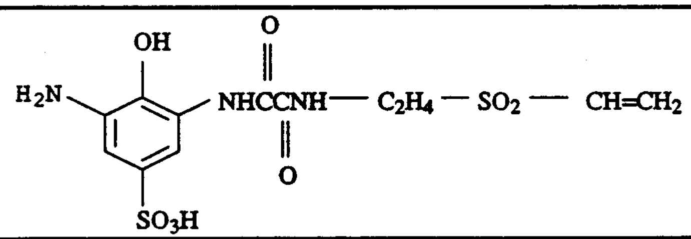 |
| 14.38 | 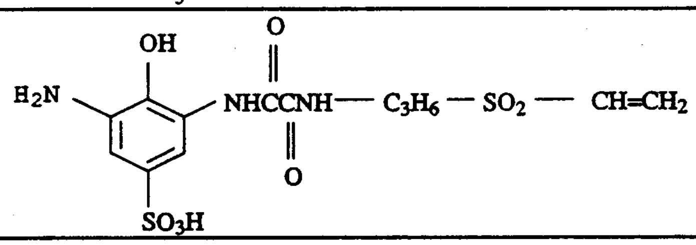 |

| Bsp.-Nr. | Formel |
|---|---|
| 14.39 | 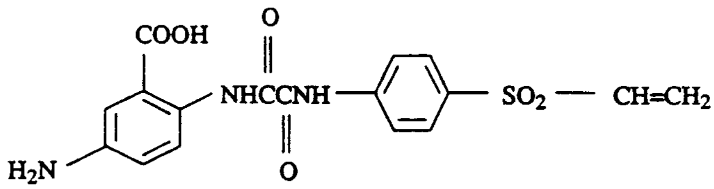 |
| 14.40 | 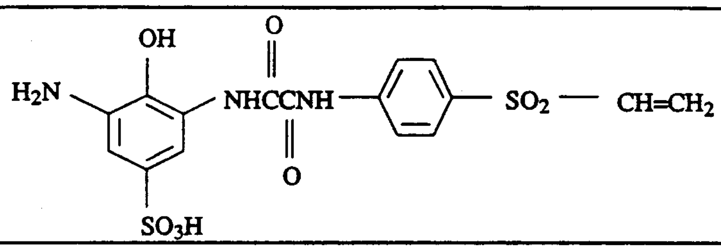 |

Beispiel 15.1

In 1000 g 23 gew.-%iges Oleum wurden bei Raumtemperatur 16 g (0,04 mol) der in Beispiel 13.1 beschriebenen Verbindung eingetragen und 20 Stunden bei Raumtemperatur gerührt. Nach beendeter Umsetzung (DC-Kontrolle) wurde das Reaktionsgemisch unter Eiskühlung auf 100 ml gesättigte Kaliumchloridlösung zerlegt. Der entstandene Niederschlag wurde abgesaugt, mit gesättigter Kaliumchloridlösung neutral gewaschen und getrocknet. Es verblieben 75 g eines Feststoffs, der neben dem Salzanteil eine Verbindung der Formel

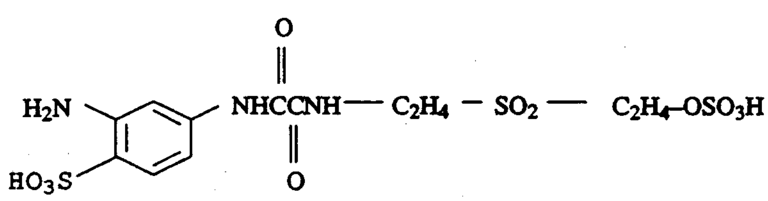

enthielt.
$^1$H-NMR ($d_6$-DMSO): $\delta$ = 3,40 (t, 2H, $CH_2$); 3,48 (t, 2H, $CH_2$); 3,70 (q, 2H, $CH_2$); 4,17 (t, 2H, $CH_2$); 7,72-8,05 (m, 3H Aromaten-H); 9,17 (t, 1H, NH); 11,05 (s, 1H, NH) ppm.

In analoger Weise werden die in der folgenden Tabelle 15 aufgeführten Verbindungen erhalten.

Tabelle 15

| Bsp.-Nr. | Formel |
|---|---|
| 15.2 | 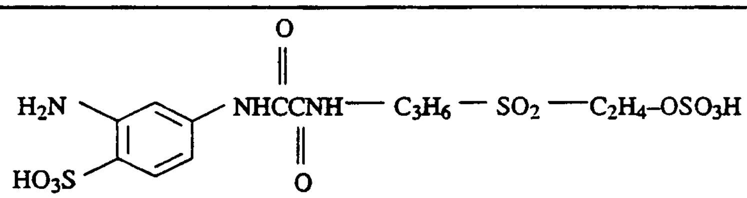 |
| 15.3 | 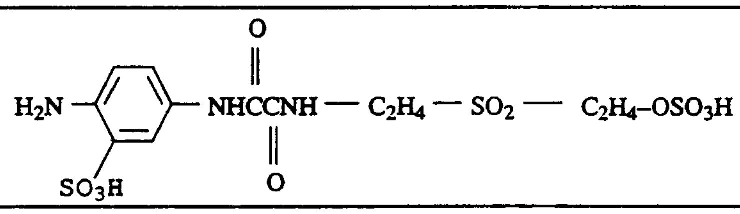 |
| 15.4 | 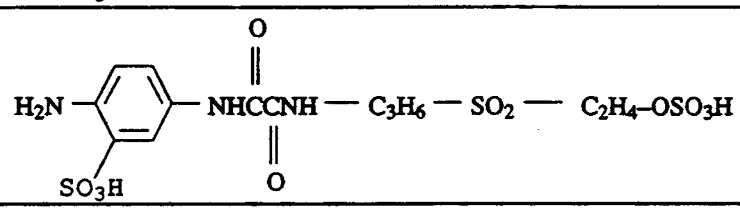 |

Beispiel 16.1

In 200 ml 1,4-Dioxan wurden 52 g (0,15 mol) der in Beispiel 3.11 beschriebenen Verbindung suspendiert, mit 10 g (0,165 mol) Ethanolamin versetzt und 2 Stunden auf 85 °C erhitzt. Nach beendeter Umsetzung (DC-Kontrolle) wurde das Reaktionsgemisch abgekühlt, der Niederschlag abgesaugt, mit t-Butylmethylether gewaschen und getrocknet. Es verblieben 40 g einer Verbindung der Formel

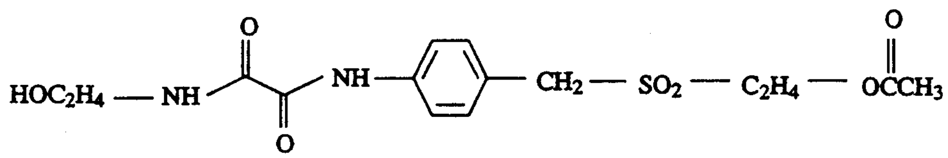

$^{13}$C-NMR ($d_6$-DMSO): $\delta$ = 20,5 ($OCOCH_3$); 42,0 ($NCH_2$); 50,5 ($SO_2$-$CH_2$-$CH_2$); 57,1 ($CH_3COOCH_2$); 58,9 ($C_6H_4$-$CH_2$-$SO_2$); 59,3 ($CH_2OH$); 120,4; 124,1; 131,1; 134,1; 137,8 (Aromaten-C); 158,6 ($C_6H_4NH\underline{C}O$); 159,9 ($NH\underline{C}O$); 169,9 ($H_3\underline{C}$-CO) ppm.

In analoger Weise werden die in Tabelle 16 aufgeführten Verbindungen erhalten.

Tabelle 16

| Bsp.-Nr. | Formel |
|---|---|
| 16.2 | 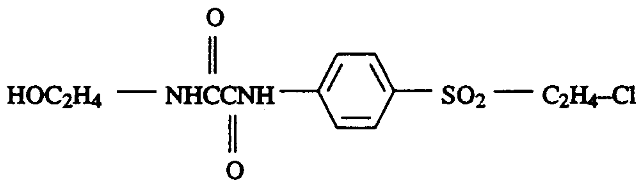 |
| 16.3 | 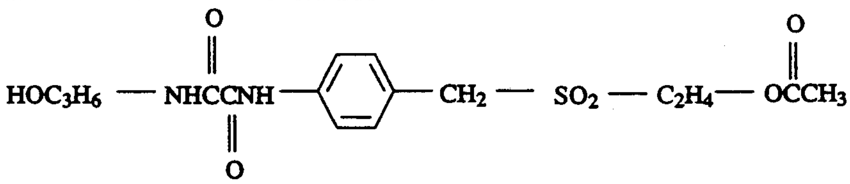 |
| 16.4 | 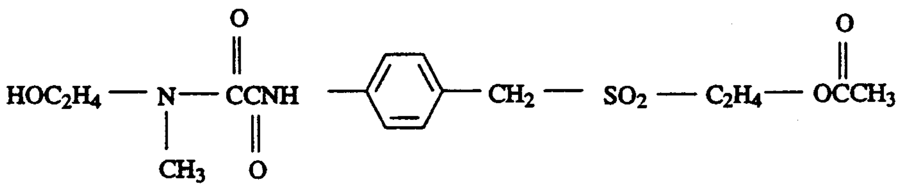 |
| 16.5 | 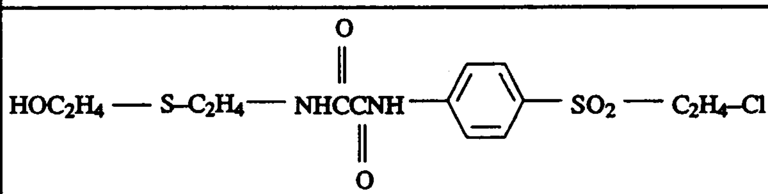 |
| 16.6 | 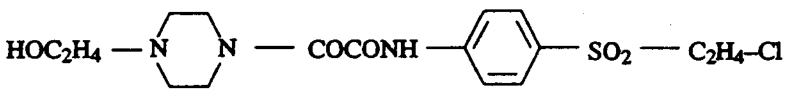 |
| 16.7 | 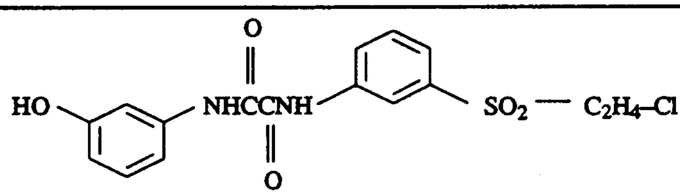 |
| 16.8 | 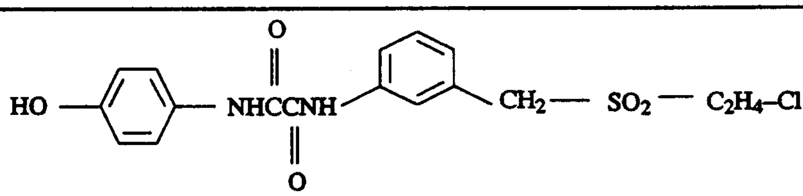 |

| Bsp.-Nr. | Formel |
|---|---|
| 16.9 | 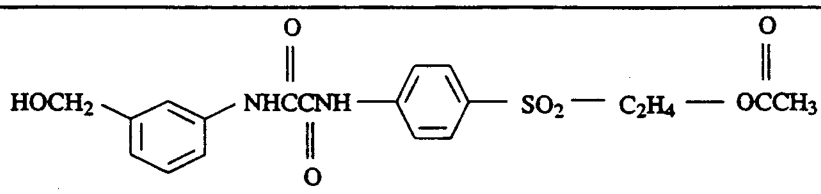 |
| 16.10 | 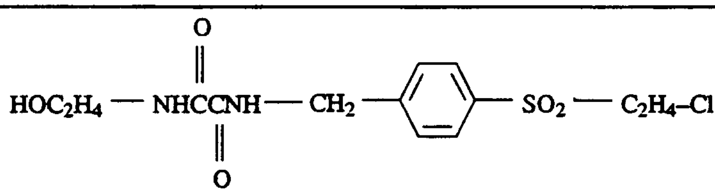 |
| 16.11 | 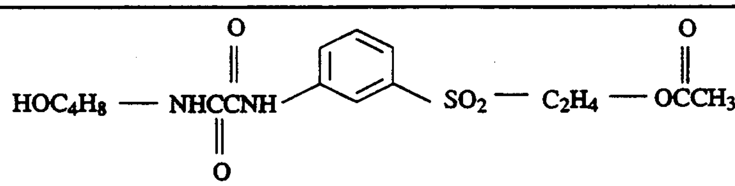 |

## Patentansprüche

1. Oxalsäurediamide der Formel I

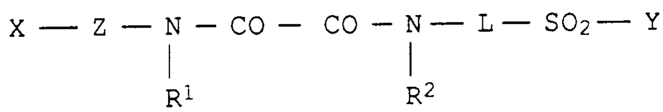 (I),

in der

- $R^1$ und $R^2$ unabhängig voneinander jeweils Wasserstoff, $C_1$-$C_4$-Alkyl oder Phenyl,
- X Hydroxy, Nitro oder einen Rest der Formel $-NR^3R^4$, worin $R^3$ für Wasserstoff oder $C_1$-$C_4$-Alkanoyl und $R^4$ für Wasserstoff, $C_1$-$C_4$-Alkyl oder Phenyl stehen,
- Z $C_2$-$C_8$-Alkylen, gegebenenfalls substituiertes Phenylen, gegebenenfalls substituiertes Naphthylen oder X-Z und $R^1$ zusammen mit dem sie verbindenden Stickstoffatom den Rest der Formel

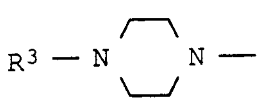 ,

worin $R^3$ die obengenannte Bedeutung besitzt,

- L $C_2$-$C_8$-Alkylen, einen Rest der Formel

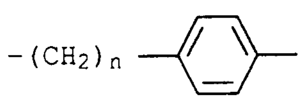 oder 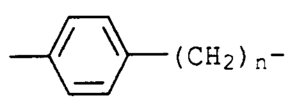 ,

worin n für 1 oder 2 steht, gegebenenfalls substituiertes Phenylen oder Naphthylen und

- Y Vinyl oder einen Rest der Formel $-C_2H_4$-A, worin A für Hydroxy oder eine unter

alkalischen Reaktionsbedingungen abspaltbare Gruppe steht, bedeuten.

2. Oxalsäurediamide nach Anspruch 1, dadurch gekennzeichnet, daß X Hydroxy oder Amino bedeutet.

3. Oxalsäurediamide nach Anspruch 1, dadurch gekennzeichnet, daß Z und L unabhängig voneinander Phenylen oder $C_2$-$C_6$-Alkylen bedeuten.

4. Oxalsäurediamide nach Anspruch 1, dadurch gekennzeichnet, daß Y Vinyl oder einen Rest der Formel -$C_2H_4$-A, worin A für eine unter alkalischen Reaktionsbedingungen abspaltbare Gruppe steht, bedeutet.

5. Verwendung von Oxalsäurediamiden gemäß Anspruch 1, die der Formel Ic

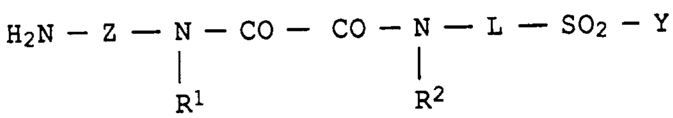

(Ic)

gehorchen, in der Z gegebenenfalls substituiertes Phenylen oder gegebenenfalls substituiertes Naphthylen und Y Vinyl oder einen Rest der Formel -$C_2H_4$-A, worin A für eine unter alkalischen Reaktionsbedingungen abspaltbare Gruppe steht, bedeuten und $R^1$, $R^2$ und L jeweils die in Anspruch 1 genannte Bedeutung besitzen, als Ankersystem und gegebenenfalls gleichzeitig als Diazokomponente oder Kupplungskomponente bei der Herstellung von Reaktivfarbstoffen.

6. Verwendung von Oxalsäurediamiden gemäß Anspruch 1, die der Formel Id

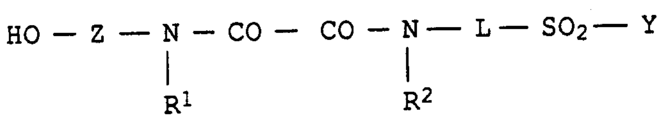

(Id)

gehorchen, in der Y Vinyl oder einen Rest der Formel -$C_2H_4$-A, worin A für eine unter alkalischen Reaktionsbedingungen abspaltbare Gruppe steht, bedeutet und $R^1$, $R^2$, Z und L jeweils die in Anspruch 1 genannte Bedeutung besitzen, als Ankersystem bei der Herstellung von Reaktivfarbstoffen.

Europäisches Patentamt

# EUROPÄISCHER RECHERCHENBERICHT

Nummer der Anmeldung

EP 93 10 0120

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 437 699 (BASF AKTIENGESELLSCHAFT)<br>* Seite 17; Anspruch 3 *<br>* Seite 6 - Seite 8; Beispiele 1,2,4-7 *<br>--- | 1-5 | C07C317/28<br>C07C317/32<br>C07C317/40<br>C07D295/18 |
| A | EP-A-0 210 951 (CIBA-GEIGY AG)<br>* Seite 55 - Seite 57; Ansprüche 14-16,19 *<br>* Seite 25 - Seite 27; Beispiele 3-9 *<br>* Seite 32 - Seite 34; Beispiele 11,12 *<br>--- | 1-5 | |
| A | EP-A-0 384 276 (HOECHST AKTIENGESELLSCHAFT)<br>* Seite 68 - Seite 69; Ansprüche 10,11,13 *<br>* Seite 14 - Seite 16; Beispiele A-D *<br>----- | 1,2,4,5 | |

RECHERCHIERTE SACHGEBIETE (Int. Cl.5)

C07C
C07D

1

Der vorliegende Recherchenbericht wurde für alle Patentansprüche erstellt

| Recherchenort | Abschlußdatum der Recherche | Prüfer |
|---|---|---|
| DEN HAAG | 07 APRIL 1993 | FINK D.G. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)